(19) **Europäisches Patentamt / European Patent Office / Office européen des brevets**

(11) **EP 2 633 078 B1**

(12) # EUROPEAN PATENT SPECIFICATION

(45) Date of publication and mention of the grant of the patent:
**13.06.2018 Bulletin 2018/24**

(21) Application number: **11793500.7**

(22) Date of filing: **26.10.2011**

(51) Int Cl.:
*C12Q 1/68* (2018.01)   *G01N 33/53* (2006.01)

(86) International application number:
**PCT/IL2011/000830**

(87) International publication number:
**WO 2012/056451 (03.05.2012 Gazette 2012/18)**

(54) **PERIPHERAL BLOOD GENE MARKERS FOR EARLY DIAGNOSIS OF PARKINSON'S DISEASE**

GENMARKER AUS PERIPHEREM BLUT ZUR FRÜHEN DIAGNOSE VON MORBUS PARKINSON

MARQUEURS GÉNIQUES DU SANG PÉRIPHÉRIQUE DESTINÉS AU DIAGNOSTIC PRÉCOCE DE LA MALADIE DE PARKINSON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2010 US 406782 P**

(43) Date of publication of application:
**04.09.2013 Bulletin 2013/36**

(73) Proprietors:
- **Mandel, Silvia A**
  34403 Haifa (IL)
- **Youdim, Moussa B.H.**
  Nesher 36864 (IL)
- **Riederer, Peter**
  97078 Wurzburg (DE)
- **Grunblatt, Edna**
  8957 Spreitenbach (CH)
- **Rabey, Jose M.**
  69121 Ramat Aviv (IL)
- **Molochnikov, Leonid**
  77401 Ashdod (IL)

(72) Inventors:
- **Mandel, Silvia A**
  34403 Haifa (IL)
- **Youdim, Moussa B.H.**
  Nesher 36864 (IL)
- **Riederer, Peter**
  97078 Wurzburg (DE)
- **Grunblatt, Edna**
  8957 Spreitenbach (CH)
- **Rabey, Jose M.**
  69121 Ramat Aviv (IL)
- **Molochnikov, Leonid**
  77401 Ashdod (IL)

(74) Representative: **Durán-Corretjer, S.L.P.**
**Còrsega, 329**
**(Paseo de Gracia/Diagonal)**
**08037 Barcelona (ES)**

(56) References cited:
**WO-A2-2005/067391**

- **CLEMENS R. SCHERZER ET AL: "Molecular markers of early Parkinson's disease based on gene expression in blood", PNAS, vol. 104, no. 3, 16 January 2007 (2007-01-16), pages 955-960, XP055019562, DOI: 10.1073/pnas.0610204104**
- **GRUNBLATT E. ET AL.: PARKINSONISM AND RELATED DISORDERS, vol. 13, no. suppl. no. 2, 1.011, 10 December 2007 (2007-12-10), page S30, XP002669982,**
- **GALTER DAGMAR ET AL: "ALDH1 mRNA: presence in human dopamine neurons and decreases in substantia nigra in Parkinson's disease and in the ventral tegmental area in schizophrenia", NEUROBIOLOGY OF DISEASE, BLACKWELL SCIENTIFIC PUBLICATIONS, OXFORD, GB, vol. 14, no. 3, 1 December 2003 (2003-12-01), pages 637-647, XP002511804, ISSN: 0969-9961, DOI: 10.1016/J.NBD.2003.09.001**
- **C ANTHONY ALTAR ET AL: "Target Identification for CNS Diseases by Transcriptional Profiling", NEUROPSYCHOPHARMACOLOGY, vol. 34, no. 1, 1 January 2009 (2009-01-01), pages 18-54, XP055019712, ISSN: 0893-133X, DOI: 10.1038/npp.2008.172**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- **EDNA GRÃ 1/4 NBLATT ET AL: "Pilot study: peripheral biomarkers for diagnosing sporadic Parkinsonâ s disease", JOURNAL OF NEURAL TRANSMISSION ; BASIC NEUROSCIENCES, GENETICS AND IMMUNOLOGY, PARKINSON'S DISEASE AND ALLIED CONDITIONS, ALZHEIMER'S DISEASE AND ADOLESCENT PSYCHIATRY RELATED DISORDERS, BIOLOGICAL PSYCHIATRY, BIOLOGICAL CHILD AND ADOLESCENT PSYCHIAT, vol. 117, no. 12, 11 November 2010 (2010-11-11), pages 1387-1393, XP019868178, ISSN: 1435-1463, DOI: 10.1007/S00702-010-0509-1**

**Description**

**TECHNICAL FIELD**

The present invention relates to the use of molecular risk marker profiles for diagnosis of Parkinson's disease. More specifically, the invention provides methods and kits for diagnosis of Parkinson's disease utilizing expression profiles of particular gene panels in blood samples.

**Abbreviations**

**ACTB,** β-actin; **AD,** Alzheimer's disease; **ALAS1,** aminolevulinate delta synthase 1; **ALDH1A1,** aldehyde dehydrogenase 1 family, member AI; **ARPP-21,** 21-cyclic AMP-regulated phosphoprotein; **CLTB,** clathrin, light polypeptide; **CNR2,** Cannabinoid receptor 2; **CSK,** c-src tyrosine kinase; **EGLN1,** egl nine homolog 1; **EIF4BP2,** eukaryotic translation initiation factor 4E binding protein 2; **GAPDH,** glyceraldehyde-3-phosphate dehydrogenase; **HIP2/UBE2K,** huntingtin interacting protein 2 / ubiquitin-conjugating enzyme E2K; **HIST1H3E,** histone cluster 1, H3e; **HSPA8/HSC70/HSC54,** chaperone heat shock 70kDa protein 8; **HS3ST2,** heparan sulfate (glucosamine) 3-O-sulfotransferase 2; **LAMB2,** laminin, β2 (laminin S); **LGALS9,** lectin, galactoside binding, soluble, 9; **LOC56920,** semaphorin sem2; **LRP6,** low density lipoprotein receptor-related protein 6; **MAN2B1,** mannosidase, alpha, class 2B, member 1; **PARVA,** parvin, alpha; **PD,** Parkinson's disease; **PENK,** proenkephalin; **PPIA,** peptidylprolyl isomerase A (cyclophilin A); **PSMA2,** proteasome (prosome, macropain) subunit, alpha type, 2; **PSMA3,** proteasome (prosome, macropain) subunit, alpha type, 3; **PSMA5,** proteasome (prosome, macropain) subunit, alpha type, 5; **PSMC4,** proteasome (prosome, macropain) 26S subunit, ATPase 4; **RPL13A,** ribosomal protein L13A; **R18S,** 18s ribosomal; **SELPLG,** selectin P ligand; **SKP1A,** S-phase kinase-associated protein 1A; **SLC31A2,** solute carrier family 31 (copper transporters), member 2; **SPHK1,** sphingosine kinase 1; **SRPK2,** SFRS protein kinase 2; **SRRM2,** serine/arginine repetitive matrix 2; **TMEFF1,** transmembrane protein with EGF-like and two follistatin-like domains 1; **TRIM36,** tripartite motif-containing 36; **UPDRS,** Unified Parkinson's Disease Rating Scale; **VMAT2,** vesicular monoamine member 2; **ZSIG11,** putative secreted protein ZSIG11.

**BACKGROUND ART**

Parkinson's disease (PD) is a progressive disorder of the central nervous system (CNS) with a prevalence of 1-2% of the adult population over 60 years of age. PD is characterized by severe motor symptoms, including uncontrollable tremor, rigidity, postural instability and slowness or absence of voluntary movement (Dauer and Przedborski, 2003). The etiology of the idiopathic form of the disease, which constitutes more than 90% of total PD cases, is still elusive, but is considered to result from both environmental and genetic factors. The clinical motor symptoms are evidently linked to the progressive degeneration of pigmented dopamine-producing neurons in the pars compacta of the substantia nigra (SNpc) (Jellinger, 2002). It is apparent that PD is a multi-system disorder involving both intra- and extra-brain areas, in which predisposed neuronal cell types in specific regions of the human peripheral, enteric and central nervous system become progressively involved (Braak *et al.,* 2006). In view of that, the pathobiological process in PD SNpc occurs later in the course of the disease, whereas other brain areas and peripheral tissues are initially affected at the pre-symptomatic phase of the disease.

Currently, the diagnosis and outcome measures of PD rest on the physician's physical examination scored with the Unified Parkinson's Disease Rating Scale (UPDRS) (Fahn and Elton, 1987) and the modified Hoehn and Yahr (H&Y) staging scale (Hoehn and Yahr, 1967). Although a diagnosis of PD can be accurately exercised in patients with a typical presentation of cardinal signs and response to levodopa treatment, the differential diagnosis *vs.* different forms of parkinsonism, e.g., essential tremor, progressive supranuclear palsy (PSP) and multisystem atrophy (MSA), may have greater overlap and thus misdiagnosis can thus occur in up to 25% of patients (Tolosa *et al.,* 2006). Imaging studies using positron emission tomography (PET) with [18F]-Dopa, single photon emission tomography (SPECT) with [123I]-β-CIT or diffusion-weighted MRI could improve differential diagnosis of Parkinsonism, but cost-effectiveness remains a problem. Yet, these tools do not provide a specific and sensitive PD diagnosis (Jankovic *et al.,* 2000). Even more frustrating is the cognizance that PD remains undetected for years before early clinical diagnosis occurs and when this happens, the loss of dopamine neurons in the substantia nigra approaches already 68% in the lateral ventral tier and 48% in the caudal nigra (Fearnley and Lees, 1991). No laboratory blood test for PD is available, let alone the detection of individuals at risk for developing PD, which is currently impossible.

Current treatment of PD is symptomatic and no truly neuroprotective drug having disease modifying activity has been developed. At present, available measures of neuroprotection are indirect and comprise functional imaging and clinical outcomes, which do not always correlate, limiting the ability to test neuroprotective drugs with disease-modifying ability. Therefore, the availability of biological markers (biomarkers) for early disease diagnosis may impact PD management in several dimensions: first, it will allow capturing individuals at high-risk before symptoms develop;

second, it will assist in discriminating between PD and similar clinical syndromes resulting from other causes. Such biomarkers, if available, may further provide a measure of disease progression that can objectively be evaluated, while clinical measures are much less accurate. Biomarkers for early PD diagnosis may help in delineating pathophysiological processes responsible for the disease, thus providing potential targets for drug intervention, and may also help in determining the clinical efficacy of new neuroprotective therapies.

In a previous large-scale transcriptomic study conducted by the inventors of the present invention in human post-mortem substantia nigra from sporadic PD patients, a number of genes with altered expression levels in brains of PD patients compared with controls have been identified. More particularly, 69 genes, e.g., LRP6, CSK, EGLN1, EIF4BP2, LGALS9, LOC56920, MAN2B1, PARVA, PENK, SELPLG, SPHK1, SRRM2 and ZS1G11 were found to have increased expression level in PD brain samples; and 68 genes, e.g., ALDH1A1, ARPP-21, HSPA8, HIP2/UBE2K, PSMC4, SKP1A, SRPK2, TMEFF1, TRIM36 and VMAT2 were found to have decreased expression level in PD brain samples (WO 2005/067391; Grünblatt et al., 2004). However, since brain samples from live patients are usually not available, in order to use this approach for diagnosing PD, it is still necessary to look for genes with altered expression patterns compared with controls in tissues such as blood, skin or saliva that can easily be obtained from living individuals.

Recent evidence has indicated that peripheral blood lymphocytes (PBL) may offer valuable surrogate markers for neuropsychiatric disorders, including bipolar disorder, schizophrenia and autism, as they share significant gene expression similarities to the more inaccessible CNS tissues (Sullivan *et al.,* 2006). However, in a following study conducted by the inventors of the present invention it was found that although the expression level of SKP1A in blood samples of PD patients is decreased compared with that in blood samples of controls, as previously found in brains samples, the expression levels of HIP2 and HSPA8, shown to be decreased in brain samples of PD patients relative to controls, are surprisingly increased in blood samples of PD patients relative to controls (Grünblatt *et al.,* 2007), indicating that the changes in the expression patterns of genes in blood of PD patients cannot always be inferred from expression pattern changes of the same genes in brain tissue of these patients.

Recent studies have shown the feasibility of studying peripheral biomarkers in cerebrospinal fluid (CSF), plasma or urine as potential diagnostics for PD (Eller and Williams, 2009). The most promising candidate in CSF appears to be alpha-synuclein, the major component of Lewy bodies whose levels are significantly lower in patients with a primary synucleopathy (idiopathic PD or dementia with Lewy bodies, DLB) (Mollenhauer *et al.,* 2008) compared to patients with Alzheimer's disease (AD) or healthy controls, though the absolute levels were very low and the test suffered from poor specificity and sensitivity. A recent study has shown that after accounting for confounding variables, such as blood CSF contamination and age, alpha-synuclein and DJ-1 protein levels were reduced in CSF from PD compared with healthy controls and AD individuals (Hong *et al.* 2010), although the test suffered from poor specificity and may have been affected by medication. In a proteomic approach-based cross sectional study aimed at identifying CSF biomarkers of PD or AD, eight potential candidates displaying a distinct pattern in both groups compared to controls were selected, but only two of them, in particular, the microtubule-associated protein tau and amylolid beta peptide 1-42, allowed for a differential diagnosis between AD and PD (Zhang *et al.,* 2008).

As for blood biomarkers, serum uric acid appears to be the first molecular factor linked to the progression of typical PD as revealed by a prospective trial showing an inverse correlation of urate levels with clinical and radiographic progression of PD (Schwarzschild *et al.,* 2008). Indeed, uric acid has been linked to a decreased risk of PD in several epidemiological studies (Weisskopf *et al.,* 2007; Davis *et al.,* 1996). In a transcriptome-wide scan study performed by Scherzer *et al.* (2007) in whole blood tissue from heterogeneous relatively early-staged PD individuals of which 80% received PD therapy, a panel of genes that may predict PD risk EDNA GRÜNBLATT ET AL: "Pilot study: peripheral biomarkers for diagnosing sporadic Parkinson's disease", JOURNAL OF NEURAL TRANSMISSION; vol. 117(12), 11.11.2010, pages 1387-1393, (i.e. after the present application's priority date) discloses a statistical model based on PSMA2, LAMP2, ALDH1A1 and HIST1H3E for the diagnosis of Parkinson's disease.

## SUMMARY OF INVENTION

As stated above, it has previously been found by the inventors of the present invention that certain genes show altered, i.e., increased or decreased, expression levels in brains of Parkinson's disease (PD) patients compared with control individuals. As later found, alterations in the expression levels of at least some of those genes relative to control individuals can also be detected in peripheral blood samples of PD patients, although not necessarily in the same direction shown in brains, and may therefore be used for diagnosis of PD in a tested individual.

The invention is defined in the claims. As found in accordance with the present disclosure certain profiles representing the normalized expression levels of particular combinations of those genes, herein also termed "gene panels", more particularly the combination of ALDH1A1, PSMC4, HSPA8, SKP1A, EGLN1 and HIP2, as well as certain combinations of three, four or five of these genes; and the combination of ALDH1A1, PSMA2, LAMB2 and optionally HIST1H3E, can differentiate with high sensitivity and specificity between PD patients, including newly diagnosed PD patients who have not received any PD therapy, and control individuals.

In one aspect, the present invention thus relates to a method for diagnosis of Parkinson's disease (PD) in a tested individual comprising determining the expression levels of genes in a blood sample of said individual, as defined in claim 1.

More particularly, the present invention relates to a computerized method for diagnosis of Parkinson's disease (PD) in a tested individual comprising analyzing, using a processor, an expression profile representing the normalized expression levels of genes in a blood sample of said individual by subjecting said expression profile to a formula based on a statistical analysis of known expression profiles, said known expression profiles representing the normalized expression level of each one of said genes in PD patients and in control individuals, thereby obtaining a value corresponding to the probability that the tested individual has PD, as defined in claim 1.

In another aspect, the present invention relates to a method for diagnosis of Parkinson's disease (PD) in a tested individual comprising determining the expression levels of genes in a blood sample of said individual, wherein said genes include ALDH1A1, PSMA2, LAMB2 and HIST1H3E.

More particularly, the present invention relates to a computerized method for diagnosis of Parkinson's disease (PD) in a tested individual comprising analyzing, using a processor, an expression profile representing the normalized expression levels of genes in a blood sample of said individual by subjecting said expression profile to a formula based on a statistical analysis of known expression profiles, said known expression profiles representing the normalized expression level of each one of said genes in PD patients and in control individuals, thereby obtaining a value corresponding to the probability that the tested individual has PD, wherein said genes include ALDH1A1, PSMA2, LAMB2 and HIST1H3E.

The statistical analysis applied to the predetermined expression profiles so as to generate the formula can be based on any suitable statistical model, e.g., a general linear model such as a logistic regression model. In particular such embodiments, the expression profile representing the normalized expression level of each one of the genes in said blood sample is subjected to the formula $P = e^N / (1 + e^N)$, wherein N represents the weighted sum of the natural logarithms of the normalized expression levels of said genes, with the addition of a constant; and P corresponds to the probability that the tested individual has PD.

In yet another aspect, the present invention provides a kit for diagnosis of Parkinson's disease (PD) in a tested individual, comprising:

(i) primers and reagents for quantitative real-time PCR amplification and measuring expression levels of the genes ALDH1A1, PSMA2, LAMB2 and HIST1H3E;
(ii) primers and reagents for quantitative real-time PCR amplification of at least one control gene for normalizing the expression levels measured in (i) to obtain normalized expression levels; and
(iii) instructions for use.

The kits of the invention are aimed at carrying out the methods defined above, and may further comprise reagents for extracting RNA from a blood sample. In certain embodiments, these kits further comprise a formula or an algorithm based on a statistical analysis of known expression profiles of the genes constituting the particular gene panel in PD patients and in controls, for applying to the normalized expression levels to obtain a value corresponding to the probability that the tested individual has PD, and said instructions include a predetermined cut-off value to which said value is compared so as to indicate whether said individual has PD.

**BRIEF DESCRIPTION OF DRAWINGS**

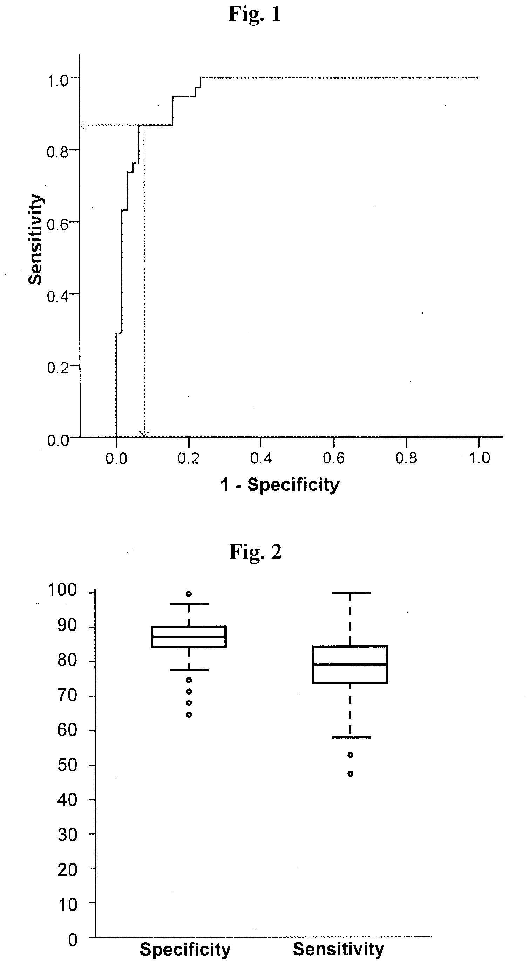

**Fig. 1** shows a receiver operating characteristic (ROC) curve of a multivariate logistic regression model based on the six genes early PD risk marker panel discriminating between *de novo* PD patients and controls. The area under the curve (AUC) is 0.96; Sensitivity represents fraction of PD patients correctly identified as such; Specificity represents fraction of control individuals correctly identified as such; and the arrows indicate the selected combination of 87% sensitivity and 92% specificity.

**Fig. 2** shows the distribution of specificity and sensitivity of the cross validation test sets as described in Example 4. Analysis of 100 randomly allocated independent test sets was performed with half of the de novo PD patients (19) and half of healthy controls (34) serving as a "training set". The resulting model was applied to the remaining de novo PD and healthy controls samples. The box plots show the median (horizontal line) and the $1^{st}$ and $3^{rd}$ quartile values (bottom and top of the box) of specificity and sensitivity (expressed as percentage). Outliers are denoted by dots.

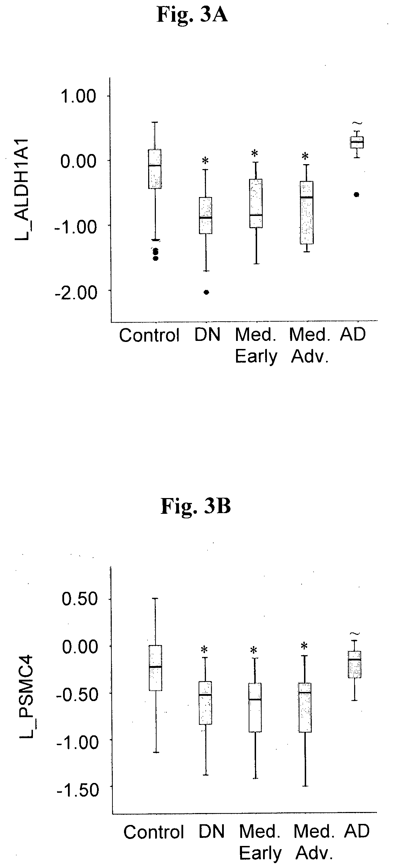

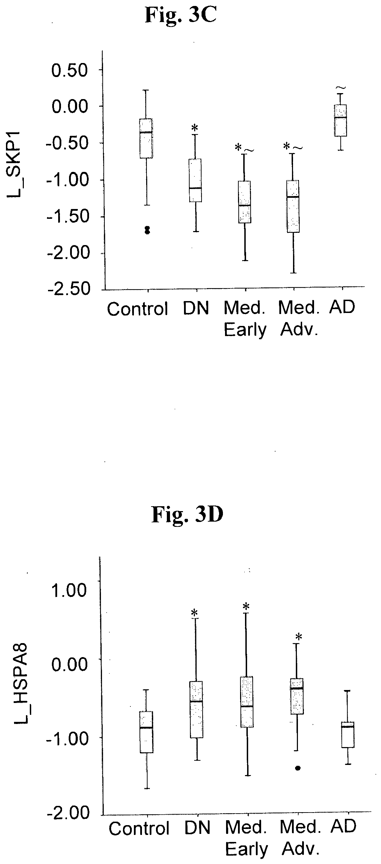

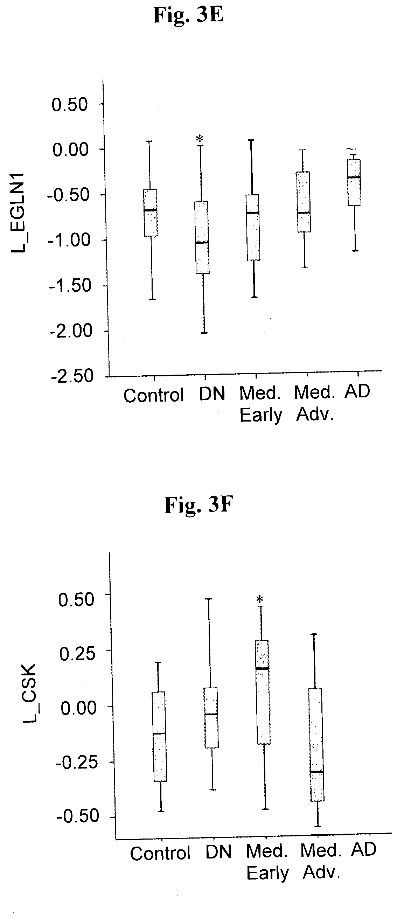

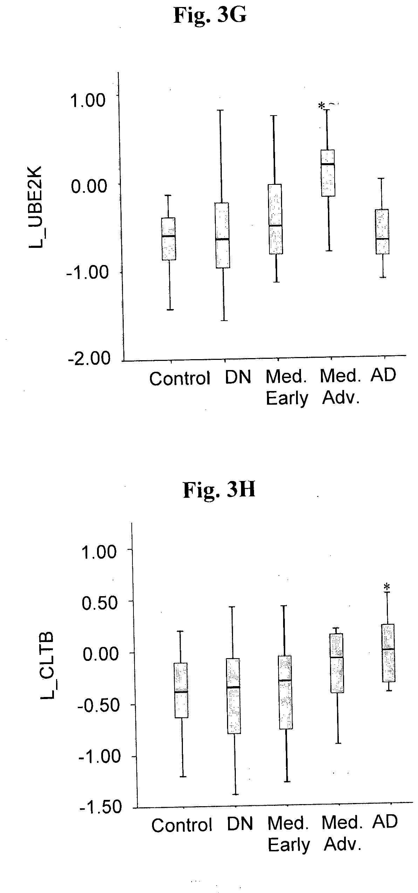

**Figs. 3A-3H** show expression levels in blood measured by quantitative RT-PCR for the eight genes used to build the PD risk marker panel, i.e., ALDH1A1 (**3A**), PSMC4 (**3B**), SKP1A (**3C**), HSPA8 (**3D**), EGLN1 (**3E**), CSK (**3F**), HIP2 (**3G**),and CLTB (**3H**). DN: *de novo* PD patients (n=38); L_ indicates the natural logarithm of the relative expression level; Med. Early: early PD patients within the first year of medication, Hoehn and Yahr (H&Y) stage 1-2 (n=24); Med. Adv.: medicated PD patients with advanced disease, H&Y stage 2.5-4 (n=16); AD: patients with AD (n=10); The box plots show the median (horizontal bold bar) and the 75th and 25th percentile values (top and bottom of the boxes) of the natural logarithms of the relative gene expression levels. The top and bottom whiskers show the lowest datum still within 1.5 interquartile range (IQR) of the lower quartile, and the highest datum still within 1.5 IQR of the upper quartile. Outliers are denoted by black dots. * denotes $p<0.05$ *vs*. the control group; ~ denotes $p<0.05$ *vs*. the DN group.

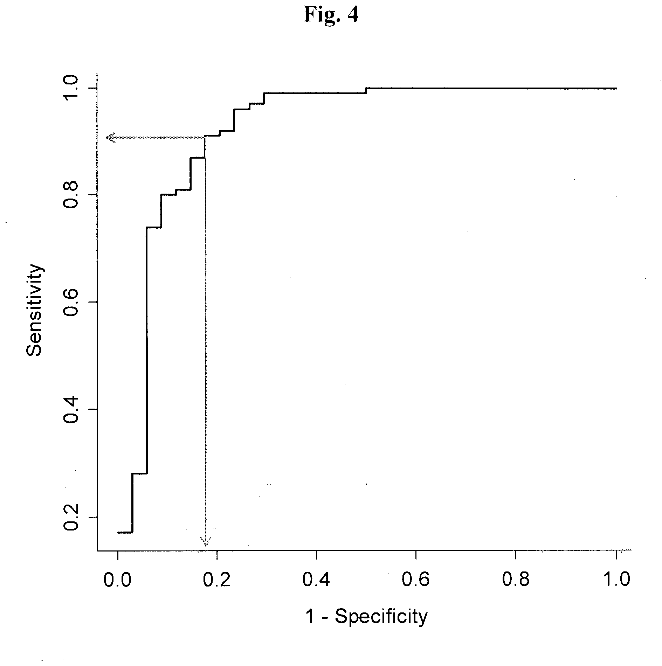

Fig. 4 shows a ROC curve of a multivariate logistic regression model based on the four genes found to discriminate between PD patients in general and controls as described in Example 7. The area under the curve is 0.92; Sensitivity and specificity are defined in Fig. 1; and the arrows indicate the selected combination of 91.5% sensitivity and 82% specificity.

## DETAILED DESCRIPTION OF THE INVENTION

The present invention provides methods for diagnosis of Parkinson's disease (PD) in a tested individual, utilizing certain profiles established based on the expression levels of certain genes, which together form a PD risk marker panel, in the peripheral blood of the individual tested, and kits for carrying out these methods. The profiles established according to the methods of the invention represent the normalized expression level of each one of the genes whose expression level is measured, i.e., the expression level of each one of the genes corrected by that of at least one control gene, in the peripheral blood of the individual tested, and are subjected to a probability equation, i.e., a predetermined formula based on a statistical analysis of known, i.e., predetermined, expression profiles representing the normalized expression level of each one of said genes in the peripheral blood of PD patients and in healthy control individuals. The outcome of this process is a value, herein also termed "probability value", ranging between 0 and 1, corresponding to the probability that the tested individual has PD.

The methods of the present invention enable discriminating PD patients from normal controls with high sensitivity and specificity. The term "sensitivity", as used herein, refers to the proportion of PD individuals, i.e., actual positives, who are correctly identified by the methods of the invention as such, and the term "specificity", as used herein, refers to the proportion of non-PD individuals, i.e., healthy individuals or individuals suffering from diseases, disorders or conditions other than PD, who are correctly identified by the methods of the invention as such. The data presented herein, including for the first time a comparison between a group of early-diagnosed PD patients who have not yet received PD therapy, i.e., *de novo* PD patients, and control individuals, show that these methods could be used with high sensitivity and specificity for PD diagnosis, especially in asymptomatic individuals or individuals at the early pre-motor stages such as patients with depression, sleep disturbances or hyposmia, or patients carrying genetic risk factors, and even for identifying individuals at risk for developing PD. As further found (data not shown), these methods are also capable of discriminating with high sensitivity and specificity between PD patients and individuals exhibiting Parkinsonian-like symptoms such as patients suffering from progressive supranuclear palsy (PSP) and multiple system atrophy (MSA).

The methods of the invention are aimed, in fact, at predicting the likelihood of PD in a tested individual, wherein an expression profile representing the expression level of each one of the genes constituting a particular gene panel in the peripheral blood of said individual is subjected to a statistical analysis, and the outcome of this process is a probability value ranging between 0 and 1, which is then used for determining, under the sensitivity and specificity limitations of the particular method used, whether said individual is positive or negative, i.e., has PD or not, respectively. The decision whether the tested individual is positive or negative is made after comparing the probability value obtained with a predetermined cut-off probability value, herein also termed "cut-off value", ranging between 0 and 1 and preferably representing the optimal combination of sensitivity and specificity as may be deduced, i.e., inferred, from the statistical analysis used. A probability value higher than the cut-off value indicates a "positive" diagnosis and a probability value lower than the cut-off value indicates a "negative" diagnosis. Although the optimal combination of sensitivity and specificity may be deduced from the statistical analysis used, the cut-off value, to a certain extent, is arbitrary and may be determined based, *inter alia,* on considerations other than optimal sensitivity and specificity, such as clinical and/or budget issues.

In view of that, it may generally be concluded that in certain cases, e.g., wherein the probability value obtained for a certain individual is either higher or lower than, but relatively close to, the cut-off value, additional diagnostic methods such as various imaging methods and CSF analyses may be recommended so as to provide as reliable a diagnosis of PD for said individual as possible.

Imaging PD involves either detecting alterations in brain structure or examining functional changes in brain metabolic systems. In PD, degeneration of the dopaminergic system is accompanied by cholinergic, noradrenergic and serotonergic dysfunction. Function of the dopaminergic and nondopaminergic systems can be imaged with positron emission tomography (PET) and single photon emission tomography (SPECT), and may be correlated with motor and non-motor disturbances. Dopa decarboxylase activity at dopamine terminals and dopamine turnover can both be measured with PET using [18F]-Dopa. Presynaptic dopamine transporters (DATs) can be followed with PET and SPECT tracers such as 123I-2β-carbomethoxy-3β-(4-iodophenyl)-N-(3-iodophenyl) tropane ([123I]-β-CIT) while vesicle monoamine transporter (VMAT) density in dopamine terminals can be examined with 11C-dihydrotetra-benazine (11C-DTBZ) PET. Measurements of dopamine terminal function can sensitively detect dopamine deficiency in both symptomatic patients and individuals at risk for Parkinsonian syndromes (Brooks, 2008), but have poor specificity for discriminating between typical (idiopathic) and atypical PD, e.g., head trauma, drug-induced Parkinsonism, PSP and MSA. On the other hand, measurements of glucose metabolism with 18F-fluorodeoxyglucose PET can be very helpful as normal or raised levels were found in the lentiform nucleus of PD but levels were reduced in MSA and PSP [Eckert *et al.,* 2007]. Magnetic resonance imaging (MRI) and transcranial sonography (TCS) can reveal brain structural changes such as volumetric reduction and hyper echogenicity of certain midbrain and striatal areas in patients with PD (Berg, 2008). They might be particularly valuable for revealing a susceptibility to PD, although they correlate less well with either clinical status or loss of dopamine terminal function in the striatum. By contrast, PET and SPECT measurements of dopamine terminal function do correlate significantly with clinical disability (Brooks, 2008). CSF analyses could differentiate between pure PD, dementive processes and infective/inflammatory processes.

Most of the genes selected for the studies underlying the methods of the invention have been chosen among the genes found to show an altered, i.e., increased or decreased, expression level in the substantia nigra of sporadic PD patients compared with substantia nigra of control individuals, as disclosed in the aforesaid WO 2005/067391, although it was already known that the directions of the alterations in brains of PD patients are not necessarily consistent with those that may be found in the peripheral blood of PD patients, as in fact shown with respect to particular two of those genes prior to these studies; and it was further realized that some of these genes may not be altered at all in peripheral blood of PD patients or that alteration thereof may not be significant.

In the limited study disclosed in WO 2005/067391, alterations in the expression levels of certain genes in brains of PD patients *vs.* controls were measured post mortem so as to find specific genes displaying differential expression levels in most of the brains tested, but no correlations were made between those genes, and no particular combination of such genes was suggested as a possible gene panel for predicting PD in a tested individual. In sharp contrast, the studies underlying the present invention were aimed at arriving at particular gene panels based on alterations in the expression levels of particular genes in the peripheral blood of PD patients *vs.* controls, wherein a combination of alterations in the expression levels of certain genes subjected to a particular statistical analysis, rather than simply an alteration in the expression level of one or more of said genes, is used for predicting the probability of PD in a tested individual, thus for diagnosing whether said individual has PD. Furthermore, since PD prediction according the methods of the present invention is based on a profile established for a panel of genes rather than an alteration in the expression level of one or more genes selected from a particular list, the genes constituting the gene panel are not necessarily those having the highest or lowest average fold change in their expression levels in PD patients relative to that of control individuals.

As shown herein, the outcome of the present studies is two partially overlapping gene panels, herein also termed "PD risk blood marker panels". The expression profiles established for the genes constituting each one of these two panels enable discriminating, i.e., distinguishing, with high sensitivity and specificity PD patients from control individuals or individuals having diseases, disorders or conditions other than PD, and thus can be used for diagnosis of PD in a tested individual.

The first gene panel disclosed herein is the outcome of the studies described in Examples 2-6 herein, and comprises at least three of the genes ALDH1A1, PSMC4, HSPA8, SKP1A, HIP2 and EGLN1, but preferably comprises all of these six genes.

As described in Examples 2-6, in order to find a gene panel which can be used for early detection and diagnosis of PD, newly diagnosed PD patients who were not undergoing dopamine treatment, i.e., *de novo* patients, were selected, as these patients represent a very early disease stage and are exempt of any potential bias on gene expression due to drug effects. The transcriptional expression level of the genes ALDH1A1, PSMC4, SKP1A, HSPA8, CSK, HIP2, EGLN1 and CLTB were assessed in blood samples obtained from said *de-novo* PD patients and from healthy age-matched controls; the relative expression level of each one of these genes was normalized; and a stepwise multivariate logistic regression analysis was then used arriving at the combination of the aforesaid six genes as an optimal predictor of PD, and at a probability equation capable of distinguishing *de novo* PD patients from controls with high degrees of sensitivity (87%) and specificity (92%). Stopping the stepwise multivariate logistic regression after finding three, four or five of the genes enabled arriving at additional panels consisting of three, four or five of these genes (ALDH1A1, PSMC4 and HSPA8; ALDH1A1, PSMC4, HSPA8 and SKP1A; and ALDH1A1, PSMC4, HSPA8, SKP1A and HIP2, respectively) capable of distinguishing *de novo* PD patients from controls with sensitivity of 79-87% and specificity of 87-90%.

Of the six genes composing this PD risk blood marker panel, the expression levels of ALDH1A1, PSMC4 and SKP1A were altered in a direction similar to that previously observed in post-mortem human substantia nigra, supporting the notion that blood signatures can serve as potential surrogate markers of PD and probably reflect relevant molecular processes occurring in PD brain. Indeed, SKP1 is a component of the E3 ligase SCF (Skp, Cullin, F-box containing complex), which together with the chaperone Hsc-70, the proteasomal ATPase subunit PSMC4, the huntingtin-interacting protein HIP2 and CLTB (a component of endocytotic vesicles mediating dopamine active transporter (DAT) internalization, are all intimately connected to dopamine metabolism and protein processing/degradation via ubiquitination and proteasomal/lysosomal-mediated degradation (Zheng *et al.,* 2010; Feldman *et al.,* 1997; Mardh and Vallee, 1986; Hjelle and Petersen, 1983; De Pril *et al.,* 2007). Ubiquitination and proteasomal-mediated protein handling defects are considered common features in PD and other chronic neurodegenerative diseases such as AD, amyotrophic lateral sclerosis (ALS) and Huntington disease (Ciechanover and Brundin, 2003; Dawson and Dawson, 2003). Further evidence for a possible functional connection between the genes included in this panel is provided by Fishman-Jacob *et al.* (2009), showing that silencing SKP1A in the substantia nigra-derived murine cell line SN4741 induced a parallel down-regulation in the transcripts of ALDH1A1 and HSPA8.

As found, the expression levels of the genes ALDH1A1, PSMC4, SKP1A and EGLN1, which were decreased in PD patients compared with controls, significantly decreased the risk for PD diagnosis, as indicated by their negative regression coefficients, whereas the expression levels of HSPA8 and HIP2, which were increased in PD patients compared with controls, significantly increased the risk for PD diagnosis.

The finding that HSPA8 and HIP2 are included in the gene panel was surprising since the direction of the alteration in their expression levels in peripheral blood of PD patients was not consistent with that previously observed in brains of PD patients. The inclusion of HIP2 in the gene panel was further surprising as the alteration in the expression level of this particular gene in *de novo* patients *vs.* controls was not significant by itself.

As a more rigorous validation of the PD risk blood marker panel as a diagnostic tool, the logistic regression model developed based on the six-gene panel obtained from the comparison between *de novo* PD patients and controls was applied to a separate cohort consisting of PD patients under medication at early and advanced disease stages. The predicted probability was calculated for each individual in the group according to the probability equation developed, displaying a high sensitivity (82.5%). High sensitivity of 70-85% was also obtained when models achieved with the partial three-, four- or five-gene panels were applied. In order to test the specificity of the various profiles, an additional group consisting of Alzheimer's disease (AD) patients were tested and as found, a specificity of 100% was obtained using each one of the three-, four-, five-, and six-gene panels.

When examining the relative quantity of each gene individually at the cross-sectional level, a similar transcriptional pattern for ALDH1A, PSMC4 and HSPA8 was demonstrated in all PD cohorts compared to normal controls, indicating that these transcripts are altered at early stages of the disease and are not affected by medication or disease progression.

The data presented herein clearly demonstrate a molecular signature in peripheral blood with ability to diagnose early PD, wherein the full six-gene panel provides the most accurate diagnosis of PD. Combined with the clinical data, this gene panel has a potential value in predicting PD and possibly in diagnosing PD prior to the stage of motor disability, such as in patients with depression, sleep disturbances or hyposmia, or patients carrying genetic risk factors. Nevertheless, in cases where considerations such as cost, time or the availability of additional information render the six-gene panel unnecessary or unaffordable, partial panels such as the three-, four- or five-gene panels described above may be used.

In a more particular aspect, the present invention relates to a computerized, i.e., computer-implemented, method for diagnosis of PD in a tested individual comprising analyzing, using a processor, an expression profile representing the normalized expression levels of genes in a blood sample of said individual by subjecting said expression profile to a formula based on a statistical analysis of known expression profiles, said known expression profiles representing the normalized expression level of each one of said genes in PD patients and in control individuals, thereby obtaining a value corresponding to the probability that the tested individual has PD, as defined in claim 1.

In certain embodiments, the expression profile representing the normalized expression levels of said genes in the blood sample of the tested individual is obtained by measuring, i.e., determining, the expression levels of said genes in said blood sample and normalizing the expression levels measured.

In certain embodiments, the value obtained following applying said formula to said expression profile is compared with a predetermined cut-off value, and said value being higher than said cut-off value indicates that the tested individual has PD.

In one embodiment, the genes whose expression levels are measured according to this method are all the six genes listed above, i.e., ALDH1A1, PSMC4, HSPA8, SKP1A, HIP2 and EGLN1, and the expression profile established represents the normalized expression levels of each one of said genes for the individual tested.

In other embodiments, the genes whose expression levels are measured according to this method are any three, four or five genes out of the above six genes, i.e., ALDH1A1, PSMC4 and HSPA8; ALDH1A1, PSMC4 and SKP1A; ALDH1A1, PSMC4 and HIP2; ALDH1A1, PSMC4 and EGLN1; ALDH1A1, HSPA8 and SKP1A; ALDH1A1, HSPA8 and HIP2; ALDH1A1, HSPA8 and EGLN1; ALDH1A1, SKP1A and HIP2; ALDH1A1, SKP1A and EGLN1; ALDH1A1, HIP2 and EGLN1; ALDH1A1, PSMC4, HSPA8 and SKP1A; ALDH1A1, PSMC4, HSPA8 and HIP2; ALDH1A1, PSMC4, HSPA8 and EGLN1; ALDH1A1, HSPA8, SKP1A and HIP2; ALDH1A1, HSPA8, SKP1A and EGLN1; ALDH1A1, SKP1A, HIP2 and EGLN1; ALDH1A1, PSMC4, HSPA8, SKP1A and HIP2; ALDH1A1, PSMC4, HSPA8, SKP1A and EGLN1; and the expression profile established represents the normalized expression levels of each one of said three, four or five genes for the individual tested.

In particular such embodiments, these genes are ALDH1A1, PSMC4 and HSPA8; ALDH1A1, PSMC4, HSPA8 and SKP1A; or ALDH1A1, PSMC4, HSPA8, SKP1A and HIP2, and the expression profile established represents the normalized expression levels of each one of said three, four or five genes, respectively, for the individual tested.

It is postulated by the inventors of the present invention that higher sensitivity and specificity of the method defined above may be achieved by adding to any of the gene panels above one or more genes whose expression level is known to be altered in blood of PD patients compared to healthy age-matched, i.e., control, individuals. Such genes may be selected, e.g., from the list of genes disclosed in the aforesaid WO 2005/067391, whose expression level is known to be altered at least in brains of PD patients.

In particular such embodiments, the genes whose expression levels are measured according to this method include ALDH1A1, PSMC4 and HSPA8; ALDH1A1, PSMC4, HSPA8 and SKP1A; ALDH1A1, PSMC4, HSPA8, SKP1A and HIP2; or ALDH1A1, PSMC4, HSPA8, SKP1A, HIP2 and EGLN1, as well as one or more additional genes such as those disclosed in WO 2005/067391, preferably one or more additional genes selected from ARPP-21, SLC18A2, SRPK2, TMEFF1, TRIM36, ADH5, PSMA3, PSMA2, PSMA5, EIF4EBP2, LGALS9, LOC56920, LRP6, MAN2B1, PARVA, PENK, SELPLG, SPHK1, SRRM2, LAMB2, HIST1H3E or ZSIG11. In more particular such embodiments, the one or more additional genes included in the expression profile established are one, two or three of the genes PSMA2, LAMB2 and HIST1H3E, more specifically, PSMA2; LAMB2; HIST1H3E; PSMA2 and LAMB2; PSMA2 and HIST1H3E; LAMB2 and HIST1H3E; or PSMA2, LAMB2 and HIST1H3E.

The second PD risk blood marker panel disclosed herein is the outcome of the studies described in Examples 7-8, and comprises the genes ALDH1A1, PSMA2, LAMB2 and HIST1H3E.

The search for candidate genes in this case, as in the study described in Examples 2-6, was based on the data previously shown by the present inventors in microarray studies with post mortem brain tissue (Grünblatt *et al.,* 2004); however, no correlation was found between the current data in blood samples and the data previously shown in post mortem brain tissue. It can be assumed that the differences observed between the current study and the previous one do not necessarily indicate a flaw, as transcription in peripheral blood cells may be altered by many factors such as copy number variations in the genome, epigenetic changes such as histone modifications, environmental changes causing biological processes such as mitochondria dysfunction, and genetic and environmental changes, or as a response to brain pathology (Hennecke and Scherzer, 2008).

As particularly described in Examples 7-8, the combination of the changes in the expression levels of these four genes gave high sensitivity and specificity indicating its potential in identifying the risk of developing PD. Whereas the expression levels of three of these genes, more specifically ALDH1A1, PSMA2 and HIST1H3E, were not influenced by PD medication, as no significant differences were observed between *de novo* PD patients and medicated PD patients, LAMB2 mRNA levels in *de novo* PD patients were significantly lower than in treated PD patients although higher compared to controls, possibly pointing to the disease progression and/or treatment effects.

As shown herein, one bias in this PD risk blood marker panel is the poor reproducibility of the HIST1H3E gene. Nevertheless, even when this gene is omitted and the expression levels of the three other genes only are used for obtaining an expression profile based on which the probability of PD is predicted, high specificity and sensitivity with AUC of 0.91 are found (data not shown). In fact, using a multiple model analysis, several models providing similar specificity and sensitivity were found, possibly indicating the complexity of PD with regard to the cause of neurodegeneration and progress, as described by Hennecke and Scherzer (2008). The selection of this particular four genes-based PD risk blood marker panel is strengthened by the specificity to sporadic PD, as no significant association was found to link the expression levels of these four genes with sporadic AD subjects.

In another aspect, the present invention thus relates to a method for diagnosis of PD in a tested individual comprising determining the expression levels of genes in a blood sample of said individual, wherein said genes include ALDH1A1, PSMA2, LAMB2 and HIST1H3E.

In a more particular aspect, the present invention relates to a computerized, i.e., computer-implemented, method for diagnosis of PD in a tested individual comprising analyzing, using a processor, an expression profile representing the normalized expression levels of genes in a blood sample of said individual by subjecting said expression profile to a formula based on a statistical analysis of known expression profiles, said known expression profiles representing the normalized expression level of each one of said genes in PD patients and in control individuals, thereby obtaining a value corresponding to the probability that the tested individual has PD, wherein said genes include ALDH1A1, PSMA2, LAMB2 and HIST1H3E.

In certain embodiments, the expression profile representing the normalized expression levels of said genes in the blood sample of the tested individual is obtained by measuring, i.e., determining, the expression levels of said genes in said blood sample and normalizing the expression levels measured.

In certain embodiments, the value obtained following applying said formula to said expression profile is compared with a predetermined cut-off value, and said value being higher than said cut-off value indicates that the tested individual has PD.

In one embodiment, the expression profile established represents the normalized expression levels of each one of said genes for the individual tested.

As in the case of the six-gene-based panel defined above, it may be assumed that higher sensitivity and specificity of this method may be achieved by adding to the gene panels above one or more genes whose expression level is known to be altered in blood of PD patients compared to control individuals such as, without being limited to, genes disclosed in WO 2005/067391. More particularly, it is postulated that higher sensitivity and specificity of this method may be achieved by adding to these gene panels one or more of the genes included in the six-gene panel desribed above, excluding ALDH1A1 *a priori* included in these gene panels, i.e., one or more of the genes PSMC4, HSPA8, SKP1A, HIP2 and EGLN1. In particular such embodiments, the one or more additional genes included in these gene panels are one, two or three of the genes PSMC4, HSPA8 and SKP1A, more specifically, PSMC4; HSPA8; SKP1A; PSMC4 and HSPA8; PSMC4 and SKP1A; HSPA8 and SKP1A; or PSMC4, HSPA8 and SKP1A.

Measuring expression levels for each one of the genes can be carried out using a variety of methods known in the art for detection and quantitating of gene products such as, without being limited to, those disclosed in detail in the experimental section hereinafter. The term "gene product" as used herein refers to the expression product, which may be either the direct transcript of the gene, i.e., an RNA such as mRNA, tRNA, or any other type of RNA, or a protein encoded by translation of a mRNA. RNA levels can be measured by appropriate methods such as nucleic acid probe microarrays, Northern blots, RNase protection assays (RPA), quantitative reverse-transcription PCR (RT-PCR), dot blot assays and *in-situ* hybridization. Alternatively, protein levels can be measured using methods based on detection by antibodies. Accordingly, the expression level of each one of the genes measured according to the methods of the present invention is, in fact, the measured level of a product expressed by each one of said genes, wherein said product may be either a protein expressed by said gene or RNA transcribed from said gene, or both.

In certain embodiments, the expression level, more particularly the amount of gene transcript, of each one of the genes is determined, i.e., quantitated, using a nucleic acid probe array. Such nucleic acid probe arrays can be of different types and may include probes of varying types such as, e.g., short-length synthetic probes (20-mer or 25-mer), full length cDNA or fragments of gene, amplified DNA, fragments of DNA (generated, e.g., by restriction enzymes) and reverse transcribed DNA. The nucleic acid probe array may be a custom array, including probes that hybridize to particular preselected subsequences of mRNA gene sequences of the genes or amplification products thereof, or a generic array designed to analyze mRNAs irrespective of sequence.

In methods using a nucleic acid probe array, nucleic acids obtained from a test blood sample are usually reverse-transcribed into labeled cDNA, although labeled mRNA can be used directly. The sample containing the labeled nucleic acids is then contacted with the probes of the array, and upon hybridization of the labeled nucleic acids that are related to the tested genes to the probes, the array is typically subjected to one or more high stringency washes to remove unbound nucleic acids and to minimize nonspecific binding to the nucleic acid probes of the arrays. Binding of labeled nucleic acid is detected using any of a variety of commercially available scanners and accompanying software programs. For example, if the nucleic acids from the sample are labeled with a fluorescent label, hybridization intensity can be determined by, e.g., a scanning confocal microscope in photon counting mode. The label can provide a signal that can be amplified by enzymatic methods, or other labels can be used including, e.g., radioisotopes, chromophores, magnetic particles and electron dense particles.

Those locations on the probe array that are hybridized to labeled nucleic acid are detected using a reader as commercially available. For customized arrays, the hybridization pattern can then be analyzed to determine the presence and/or relative or absolute amounts of known mRNA species in the sample being analyzed.

In other embodiments, the expression levels, more particularly the gene transcript, of each one of the genes is quantitated using a real time reverse-transcription PCR (real time RT-PCR) method, as exemplified herein. These methods involve measurement of the amount of amplification product formed during an amplification process, e.g., by a fluorogenic nuclease assay, to detect and quantitate specific transcripts of the genes of interest. These assays continuously measure PCR product accumulation using a dual-labeled fluorogenic oligonucleotide probe as in the approach frequently referred to in the literature simply as the TaqMan® method.

The probe used in real time PCR assays is typically a short (ca. 20-25 bases) polynucleotide labeled with two different fluorescent dyes, i.e., a reporter dye at the 5'-terminus of the probe and a quenching dye at the 3'-terminus, although the dyes can be attached at other locations on the probe as well. For measuring a specific transcript, the probe is designed to have at least substantial sequence complementarity with a probe binding site on the specific transcript. Upstream and downstream PCR primers that bind to regions that flank the specific transcript are also added to the reaction mixture for use in amplifying the nucleic acid.

When the probe is intact, energy transfer between the two fluorophores occurs and the quencher quenches emission from the reporter. During the extension phase of PCR, the probe is cleaved by the 5'-nuclease activity of a nucleic acid polymerase such as Taq polymerase, thereby releasing the reporter dye from the polynucleotide-quencher complex and resulting in an increase of reporter emission intensity that can be measured by an appropriate detection system. The fluorescence emissions created during the fluorogenic assay is measured by commercially available detectors that comprise computer software capable of recording the fluorescence intensity of reporter and quencher over the course of the amplification. These recorded values can then be used to calculate the increase in normalized reporter emission intensity on a continuous basis and ultimately quantify the amount of the mRNA being amplified.

In further embodiments, the expression level, more particularly the amount of gene transcript, of each one of the genes is quantitated using a dot blot assay and *in-situ* hybridization. In such assays, a blood sample from the tested individual is spotted on a support, e.g., a filter, and then probed with labeled nucleic acid probes that specifically hybridize with nucleic acids derived from one or more of the genes the expression level of which is measured. After hybridization of the probes with the immobilized nucleic acids on the filter, unbound nucleic acids are rinsed away and the presence of hybridization complexes is detected and quantitated on the basis of the amount of labeled probe bound to the filter.

In certain embodiments, the gene product the level of which is measured is a protein that can be detected by an antibody or a fragment thereof, capable of binding to that protein. The antibody or fragment thereof may be detectably labeled with any appropriate marker, e.g., a radioisotope, an enzyme, a fluorescent label, a paramagnetic label, or a free radical.

According to the methods of the present invention, normalization of the expression levels measured for each one of the genes is carried out by correcting the measured expression level of each one of said genes by the expression level of at least one control, i.e., reference, gene whose expression in blood is relatively stable. Examples of control genes that may be used according to these methods include, without being limited to, R18S, ACTB, ALAS1, GAPDH, RPL13A and PPIA. In certain embodiments, normalization of the expression levels measured for each one of the genes is carried out by dividing the expression level measured for each of said genes by the geometric mean of the expression levels of more than one, i.e., two, three, four or more, control genes.

The known expression profiles used according to the methods of the present invention are predetermined expression profiles representing the normalized expression level of each one of the genes measured in PD patients and in control individuals. A statistical analysis is applied to these predetermined expression profiles, using a processor, so as to generate a formula, which can then be applied to the expression profile established representing the normalized expression level of each one of the genes for the tested individual. The end result of subjecting to that formula the expression profile of the tested individual is a value between 0 and 1 corresponding to the probability that said individual has PD, which is compared to a cut-off value to determine a positive or negative diagnosis.

The term "processor", as used herein, refers to a logic circuitry that responds to and processes the basic instructions that drive a computer system. A processor may also be implemented as a microprocessor, microcontroller, application specific integrated circuit (ASIC) or discrete logic.

The statistical analysis applied to the predetermined expression profiles in order to generate the formula can be based on any suitable statistical model. In certain embodiments, the statistical model is a general linear model, such as a logistic regression model or classification trees. According to a more particular embodiment, the statistical model is a logistic regression model.

In particular embodiments, the statistical model is a logistic regression model, and the expression profile representing the normalized expression level of each one of the genes whose expression levels are measured for the tested individual is subjected to the formula $P = e^N / (1+e^N)$, wherein N represents the weighted sum of the natural logarithms of the normalized expression levels of said genes, with the addition of a constant, calculated by summing the natural logarithms of all of the normalized expression levels included in the expression profile established, each multiplied by a predetermined regression coefficient value, and adding a predetermined constant value; and P is a value between 0 and 1 corresponding to the probability that the tested individual has PD. It should be noted that the predetermined regression coefficient values used to multiply the natural logarithm of each one of the normalized expression levels included in the expression profile established, as well as the predetermined constant added, are determined by the statistical analysis used so as to generate the formula.

In view of the experimental data shown in Examples 2-6, in one specific such embodiment, the expression profile established represents the normalized expression levels of the genes ALDH1A1, PSMC4, HSPA8, SKP1A, HIP2 and EGLN1 in a blood sample of said individual, and said expression profile is subjected to the formula:

$$P = e^N / (1+e^N),$$

wherein $N = -2.078 + \sum_{i=1-6} (B_i \cdot 10 \cdot \ln(\text{Gene\_exp}_i))$; each i in said formula indicates a different gene i out of the following six genes: ALDH1A1, PSMC4, HSPA8, SKP1A, HIP2 and EGLN1; $B_i$ is the regression coefficient value of said gene i; Gene\_exp$_i$ is the relative expression level of said gene i in said individual; B(ALDH1A1) is -0.220; B(PSMC4) is -0.306; B(HSPA8) is 0.435; B(SKPIA) is -0.261; B(HIP2) is 0.242; B(EGLN1) is -0.190; and P corresponds to the probability that the tested individual has PD.

In another specific such embodiment, the expression profile established represents the normalized expression levels of the genes ALDH1A1, PSMC4, HSPA8, SKP1A and HIP2 in a blood sample of said individual, and said expression profile is subjected to the formula:

$$P = e^N / (1+e^N),$$

wherein N=-0.475+$\sum_{i=1-5}$ (B$_i$·10·ln(Gene_exp$_i$)); each i in said formula indicates a different gene i out of the following five genes: ALDH1A1, PSMC4, HSPA8, SKP1A and HIP2; B$_i$ is the regression coefficient value of said gene i; Gene_exp$_i$ is the relative expression level of said gene i in said individual; B(ALDH1A1) is -0.191; B(PSMC4) is - 0.354; B(HSPA8) is 0.411; B(SKPIA) is -0.236; B(HIP2) is 0.204; and P corresponds to the probability that the tested individual has PD.

In still another specific such embodiment, the expression profile established represents the normalized expression levels of the genes ALDH1A1, PSMC4, HSPA8 and SKP1A in a blood sample of said individual, and said expression profile is subjected to the formula:

$$P = e^N / (1+e^N),$$

wherein N=-0.818+$\sum_{i=1-4}$ (B$_i$·10·ln(Gene_exp$_i$)); each i in said formula indicates a different gene i out of the following four genes: ALDH1A1, PSMC4, HSPA8 and SKP1A; B$_i$ is the regression coefficient value of said gene i; Gene_exp$_i$ is the relative expression level of said gene i in said individual; B(ALDH1A1) is -0.178; B(PSMC4) is -0.284; B(HSPA8) is 0.438; B(SKP1A) is -0.182; and P corresponds to the probability that the tested individual has PD.

In yet another specific such embodiment, the expression profile established represents the normalized expression levels of the genes ALDH1A1, PSMC4 and HSPA8 in a blood sample of said individual, and said expression profile is subjected to the formula:

$$P = e^N / (1+e^N),$$

wherein N=0.176+$\sum_{i=1-3}$ (B$_i$·10·ln(Gene_exp$_i$)); each i in said formula indicates a different gene i out of the following three genes: ALDH1A1, PSMC4 and HSPA8; B$_i$ is the regression coefficient value of said gene i; Gene_exp$_i$ is the relative expression level of said gene i in said individual; B(ALDH1A1) is -0.239; B(PSMC4) is -0.322; B(HSPA8) is 0.435; and P corresponds to the probability that the tested individual has PD.

The tested individual according to any one of the methods of the present invention may be any individual suspected of having PD such as an individual exhibiting Parkinsonism or Parkinsonian-like symptoms, either already receiving PD therapy or not. The term "PD therapy" as used herein refers to any type of medical, i.e., therapeutic, treatment directed at treating PD or the symptoms thereof, including, e.g., L-Dopa (L-3,4-dihydroxyphenylalanine), dopamine agonists such as bromocriptine, pergolide, pramipexole, ropinirole, piribedil, cabergoline, apomorphine and lisuride, and monoamine oxidase (MAO)-B inhibitors such as selegiline and rasagiline, administration. In one particular embodiment, the tested individual according to the methods of the invention is an individual exhibiting Parkinsonism who has not received PD therapy, such as a *de novo* patient. In another particular embodiment, the tested individual according to these methods is an individual exhibiting Parkinsonian-like symptoms who has previously been diagnosed as either a familial or sporadic PD patient thus receiving PD therapy, i.e., a medicated PD patient, either an early medicated patient within the first year of medication or an advanced medicated patient having an advanced disease.

As described above, PD risk blood marker panels having a predictive/diagnostic potential as disclosed herein may guide highly sensitive and specific patient selection, enabling distinguishing with high sensitivity and specificity between PD patients, in particular early PD patients or individuals at high risk for developing PD, and individuals exhibiting Parkinsonian-like symptoms which are frequently inaccurately diagnosed as PD patients, such as patients suffering from PSP and MSA. Furthermore, such PD risk marker profiles may guide rational design of neuroprotective/disease modifying trials in PD with agents targeting mechanisms that are common to the particular genes included in the profile.

In yet another aspect, the present invention provides a kit for diagnosis of PD in a tested individual, comprising:

(i) primers and reagents for quantitative real-time PCR amplification and measuring expression levels of the genes ALDH1A1, PSMA2, LAMB2 and HIST1H3E;

(ii) primers and reagents for quantitative real-time PCR amplification of at least one control gene for normalizing the expression levels measured in (i) to obtain normalized expression levels; and

(iii) instructions for use.

The kits of the present invention can be used for carrying out the methods defined above, i.e., for diagnosis of PD in a tested individual utilizing any one of the PD risk marker panels described above.

As described above, in all of these methods, the expression level of each one of the genes constituting the gene panel is measured in a blood sample obtained from the tested individual, and is then normalized by the expression level measured for one or more, e.g., two, three or four, control gene so as to obtain an expression profile representing the normalized expression level of each one of the genes included in the PD risk marker panel.

The kits of the invention thus comprise both a list of genes, including one or more control genes, whose expression levels in the peripheral blood of the tested individual are determined, together with primers and reagents for quantitative real-time PCR amplification and determining the expression levels of said genes. The isolation of peripheral mononuclear cells (PMCs) from the blood sample obtained from the tested individual as well as the extraction of total RNA from said PMCs, may be carried out using any suitable technology known in the art, e.g., as described in Materials and Methods hereinafter. Examples of materials and tools that may be useful for these purposes include anticoagulants such as ethylenediaminetetraacetic acid (EDTA) and EDTA-coated tubes, materials that may be used for blood separation such as Ficoll (Sigma); and RNA extraction reagents such as TriReagent (Sigma). Measuring of the expression levels of each one of the genes of interest can be carried out by any suitable technology known in the art for detection and quantitating of gene products such as those described above, e.g., using real-time quantitative reverse transcribed PCR, as exemplified herein.

The primers provided as a part of the kit of the present invention are, in fact, oligonucleotides that can be used for the detection of said genes expressed in PMCs, wherein each one of said primers is complementary to a specific sequence in one of said genes. The primers provided may be any suitable primers enabling the detection of the specific genes the expression levels of which are measured. Non-limiting examples of oligonucleotide primers complementary to specific sequences of the genes ALDH1A1, PSMC4, HSPA8, SKP1A, HIP2, EGLN1, PSMA2, LAMB2 and HIST1H3E, R18S, ACTB, ALAS1, GAPDH, RPL13A and PPIA are provided in the Materials and Methods section hereinafter.

As described above, in order to complete the diagnosis process according to the methods of the invention, the expression profile representing the normalized expression level of each one of the genes constituting the gene panel is subjected to a predetermined formula generated based on a statistical analysis, so as to obtain a probability value corresponding to the probability that the tested individual has PD and, after comparing with a cut-off value, indicating whether the tested individual has PD or not. In certain embodiments, the kits of the invention thus further comprise a predetermined formula generated based on a statistical analysis of known expression profiles of the genes whose expression level is measured in PD patients and in control individuals, for applying to the normalized expression levels to obtain a value corresponding to the probability that the tested individual has PD; wherein the instructions for use comprised within said kit include a predetermined cut-off value to which said value is compared so as to determine a positive or negative diagnosis.

The invention will now be illustrated by the following non-limiting examples.

## EXAMPLES

### Materials and Methods for Examples 1-6

### *Study cohort*

Blood samples taken from a total of 152 individuals including 38 newly diagnosed PD patients before treatment (*de novo,* non medicated PD group); 24 early PD patients within the first year of medication (Hoehn and Yahr, H&Y, 1-2); 16 PD patients with advanced disease (H&Y 2.5-4); 10 patients diagnosed with AD; and 64 healthy age-matched controls without personal or family history of neurodegenerative diseases, were recruited. Blood samples of PD patients and controls were recruited from medical centers in Pisa and Camaiore (Italy), and from Assaf ha-Rofeh and Rambam Medical Centers (Israel); and blood samples of AD patients were recruited by the Clinic for Psychiatry, Psychotherapy and Psychosomatic, University of Würzburg. PD patients were diagnosed by neurology-board-certified movement disorders specialists that met modified United Kingdom Parkinson's Disease Society Brain Bank (Hughes *et al.,* 1992) clinical diagnostic criteria. Patient data including age, gender, PD severity score, H&Y and medication were registered and are presented in **Table 1A.** The mental scores of the AD patients recruited are presented in **Table 1B.** The proportion of males in the healthy population was 43.75% with mean age of $65.91\pm7.89$, and in the PD group (*de novo* and medicated) 65.38% with mean age of $65.91\pm10.29$. Total white blood cells count, as well as differential blood cell counts were examined for any bias in gene expression changes. No significant variations were observed via one-way ANOVA between the experimental groups in all counts (data now shown).

**Table 1A:** Demographics, H&Y scores and medication of all cohorts

| Diagnostic groups (n) | Age (SD) | Gender (%male) | H&Y (SD) | LD (%) | S/R (%) |
|---|---|---|---|---|---|
| Control (64) | 65.91 (7.89) | 43.75 | 0 | 0 | 0 |
| PD total (78) | 65.91 (10.29) | 65.38 | 1.83 (1.00) | 28 (35.9) | 19 (24.36) |
| PD de novo (38) | 62.68 (10.07) | 68.42 | 1.30 (0.62) | 0 | 0 |
| PD early (24) | 67.33 (9.95) | 62.50 | 1.58 (0.41) | 12 (50.0) | 15 (62.5) |
| PD late (16) | 71.44 (8.93) | 62.50 | 3.44 (0.60) | 16 (100.0) | 4 (25.0) |
| AD (10) | 73.3 (8.81) | 50.00 | 0 | 0 | 0 |
| SD: standard deviation; LD: L-Dopa/dopamine agonist; S/R: selegilne/rasagiline; early/late indicates medicated, early/advanced stage PD. | | | | | |

**Table 1B:** Average mental scores of AD cohort

| MMSE (SD) | DSM (SD) | UPDRS (SD) | Hamilton depression scale (SD) |
|---|---|---|---|
| 19.2 (4.8) | 2.0 (0) | 11.6 (11.48) | 3.1 (3.14) |
| MMSE1: Mini-mental state examination; DSM: Diagnostic and statistical manual of mental disorders; SD: standard deviation. | | | |

### Isolation and purification of total RNA from blood samples

Venous blood samples were collected from PD and healthy age-matched controls (pure baseline), using PAXgene Blood RNA System Tubes (Becton Dickinson GmbH, Heidelberg, Germany). These tubes contain a stabilization reagent which protects RNA molecules from degradation by RNases enabling collection, stabilization, storage and transportation of human whole blood specimens. The blood samples were frozen at -80°C until processed for total RNA isolation.

Total RNA was extracted from whole blood with the PAXgene™ Blood RNA Kit 50 (PreAnalytiX, Qiagen and BD, Germany) and spectrophotometrically scanned to assess RNA integrity and concentration using NanoDrop 1000 Spectrophotometer (Thermo Fisher Scientific Inc.).

### Quantitative Real-Time RT-PCR

Total RNA from each blood sample was reverse transcribed employing the High-Capacity cDNA Reverse Transcription Kit (Applied Biosystems, Foster City, CA, USA). Quantitative real-time RT-PCR was performed using SYBR Green detection chemistry, in the ABI PRISM 7000 Real-Time Sequence Detection System (Applied Biosystems, Foster City, CA, USA) in 96 format. Reactions were primed using QuantiTect Primer Assay, (QIAGEN, Hilden, Germany) and SYBR® Premix Ex Taq™, ROX™ Reference Dye II (Takara, Otsu, Shiga, Japan). The list of primers used is provided in **Table 2.**

The thermal cycler program consisted of an initial denaturation at 95°C for 10 minutes followed by 40 cycles of denaturation at 95°C for 15 seconds and primer annealing at 60°C for 1 minute. The results were analyzed using 7000 System SDS Software (Applied Biosystems). The threshold value. i.e., the cycle number at which the increase in fluorescence and thus cDNA is exponential, for each primer, was set manually (~0.2 for most genes). Baseline values were manually set for each primer to neutralize nonspecific background noise and were deduced from the Rn *vs.* cycle number. Rn is the fluorescence of the reporter dye (SYBR Green) divided by the fluorescence of the passive reference dye, ROX. The latter does not participate in the 5' nuclease reaction providing an internal reference for background fluorescence emission. Raw Ct value, i.e., the point at which the fluorescence crosses the threshold, was automatically transformed to quantity using the mentioned software via the equation [$Qty=10^{(ct-Intercept)/Slop}$], with Slope and Intercept values taken from the Ct *vs.* Log Qty standard curve. The resulting data were analyzed using Excel spreadsheet. In order to account for inter-assay variations, a set of at least 2 reference cDNA samples were run per plate, producing internal positive control (IPC) values, and the quantities were then normalized to IPC to control for inter-plate variability.

Table 2: Real-time PCR oligonucleotide primers

| Gene symbol | Ex. 2-6* | Ex. 7-8* | Accession number | QIAGEN Catalog No. |
|---|---|---|---|---|
| EGLN1 | √ | | NM_022051 | QT01021454 |
| HSPA8 | √ | √ | NM_006597, NM_153201 | QT00030079 |
| PSMC4 | √ | | NM_006503 | QT00035511 |
| CLTB | √ | | NM_001834 | QT00081872 |
| ALDH1A1 | √ | √ | NM_000689 | QT00013286 |
| SKP1A | √ | | NM_006930 | QT00040320 |
| HIP2/UBE2K | √ | √ | NM_005339, NM_001111113 | QT00010276 |
| CSK | √ | √ | NM_004383, NM_001127190 | QT00999131 |
| PSMA2 | | √ | NM_ 002787 | QT 00047901 |
| PSMA3 | | √ | NM_ 002788 | QT 00057344 |
| PSMA5 | | √ | NM_ 002790 | QT 00071995 |
| HS3ST2 | | √ | NM_006043 | QT 00205156 |
| SLC31A2 | | √ | NM_ 001860 | QT 00006629 |
| LAMB2 | | √ | NM_ 002292 | QT 00050771 |
| CNR2 | | √ | NM_001841 | QT 00012376 |
| HIST1H3E | | √ | NM_003532 | QT 00217896 |
| **Control genes** | | | | |
| ACTB | √ | √ | NM_ 001101 | QT00095431 |
| ALAS 1 | √ | √ | NM_000688, NM 199166 | QT00073122 |
| GAPDH | √ | √ | NM_002046 | QT01192646 (Ex. 2-6) QT00079247 (Ex. 7-8) |
| PPIA | √ | √ | NM_021130 | QT01866137 (Ex. 2-6) QT01669542 (Ex. 7-8) |
| RPL13A | √ | √ | NM_012423 | QT00089915 |
| R18S | | √ | V01270 | QT00199367 |
| * Ex. 2-6 and Ex. 7-8 represent Example 2-6 and 7-8 herein, respectively. | | | | |

### Statistical Analysis

The natural logarithms of the relative gene expression values of blood cells counts were calculated in order to induce normal distribution. Comparison between the experimental groups was carried out using one-way analysis of variance (ANOVA) followed by Tukey post hoc correction. Age and gender variables were tested using t-test and Mann-Whitney non-parametric test, respectively. Correlations were evaluated via Pearson Correlation with two tailed test of significance. In all tests, probability values of $p<0.05$ were considered statistically significant.

A logistic regression model was built via stepwise multivariate logistic regression analysis of the natural logarithms of the relative gene expression for all genes, comparing the PD *de novo* subjects and the healthy age-matched controls. Variables with significance of $p<0.05$ were accepted into the logistic regression, wherein the most significant variable is added in each step. The model was used to calculate the predicted probability for PD. A Receiver Operating Characteristic (ROC) curve was built for the predicted probability for PD and the area under the ROC curve (AUC) was calculated. All statistical analyses were performed using SPSS Statistics 17.0 software (SPSS Inc., Chicago, Illinois, USA).

**Materials and Methods for Examples 7-8**

*Study cohort*

Patients with sporadic PD (patients with familial PD were excluded by family anamnesis) and healthy elderly controls without neurological disorders or dementia were assessed. As an additional control of another neurodegenerative disease, patients with AD who did not suffer from any other neurological disorders were recruited. All subjects underwent formal diagnostic procedure according to the UK Brain Bank criteria for PD (Hughes *et al.,* 2002). The H&Y staging was used for clinical evaluation of PD (Hoehn and Yahr, 1967), and the National Institute of Neurological and Communicative Disorders and Stroke-Alzheimer's Disease and Related Disorders Association criteria (McKhann *et al.,* 1984) for AD. AD assessment scale cognitive subscale (Wouters *et al.* 2008), mini-mental state examination (MMSE) (O'Connor *et al.,* 1989), clinical dementia rating (CDR) (Berg, 1988), UPDRS (Fahn and Elton, 1987), and Hamilton depression scale (Hamilton, 1960) were administered to all subjects. In addition, detailed information on medication and smoking habits were collected. Some of the subjects were retested in a second recruitment 3-6 months after the first recruitment.

One hundred and fifty-three subjects (66 female and 87 male) with a mean age of $63.03 \pm 11.07$ years participated in the first recruitment, of which 105 had PD (age $60.5 \pm 10.7$ years, MMSE scores $28.50 \pm 2.07$, UPDRS scores $31.34 \pm 18.82$, Hamilton depression scores $2.01 \pm 3.86$); 14 had AD (age $70.8 \pm 10.2$ years, MMSE scores $18.64 \pm 6.06$, UPDRS scores $9.36 \pm 10.40$, Hamilton depression scores $3.50 \pm 3.67$); and 34 were healthy controls (age $67.6 \pm 9.8$ years, MMSE scores $29.47 \pm 1.13$, UPDRS scores $0.91 \pm 1.90$, Hamilton depression scores $3.68 \pm 6.79$). Of the 105 PD subjects, 11 were *de novo* PD subjects (age $55.7 \pm 11$ years, MMSE scores $29.2 \pm 0.87$, UPDRS scores $27 \pm 7.7$, Hamilton depression scores $4.2 \pm 7$). PD patients were treated with anti-parkinsonian standard therapy: with L-dopa and decarboxylase inhibitors as basic treatment. Sixty-seven subjects (37 female and 30 male) with a mean age of $65.67 \pm 10$ years were reinvestigated in a second recruitment, of which 22 had PD (age $61.4 \pm 9.3$ years, MMSE scores $28.68 \pm 1.21$, UPDRS scores $17.59 \pm 16.48$, Hamilton depression scores $3.09 \pm 2.83$); 12 had AD (age $69.3 \pm 10.3$ years, MMSE scores $18.00 \pm 6.47$, UPDRS scores $8.67 \pm 10.33$, Hamilton depression scores $3.17 \pm 3.43$); and 33 were healthy controls (age $67.2 \pm 9.6$ years, MMSE scores $29.55 \pm 0.94$, UPDRS scores $2.15 \pm 5.51$, Hamilton depression scores $3.33 \pm 6.11$).

*Total RNA Extraction*

Total RNA was prepared with the PAXgene™ Blood RNA Kit 50 (PreAnalytiX, Qiagen and BD, Germany). RNA isolation reagents were prepared from 0.2 1M filtered, diethyl pyrocarbonate (DEPC)-treated water (Fermentas Inc., Hanover, MD, USA) throughout the isolation procedure. Total RNA samples were spectrophotometrically scanned (Experion, BioRad Co., Hercules, CA, USA) from 220 to 320 nm; the A260/A280 of total RNA was typically> 1.9.

*Quantitative Real-Time RT-PCR*

Quantitative real-time RT-PCR was conducted for the 12 genes listed in **Table 2.** Total RNA (500 ng) from each blood sample was reverse transcribed with the random hexamer and oligo-dT primer mix using iScript (BioRad Co., Hercules, CA, USA). Quantitative real-time PCR was performed in the iCycler iQ system (BioRad Co., Hercules, CA, USA) as previously described (Grünblatt *el al.,* 2007). The genes were normalized to the six reference genes R18S, ACTB, ALAS1, GAPDH, RPL13A and PPIA according to GeNorm (Vandesompele *et al.,* 2002). Real-time PCR was subjected to PCR amplification as previously described (Grünblatt *et al.,* 2009). All PCR reactions were run in duplicate. The amplified transcripts were quantified using the comparative CT method analyzed with the BioRad iCycler iQ system program. The same procedure was used for baseline samples as well as the follow-up confirmation study. Data were analyzed with Microsoft Excel 2000 to generate raw expression values.

*Statistical analysis*

For the first recruitment, mean, standard deviation, median, minimum and maximum values were calculated for the continuous variables. For the data of the first recruitment, univariate logistic regression analyses were calculated for all the genes and the factors gender, age, CDR, MMSE, UPDRS, and Hamilton depression scale scores comparing the diagnosed PD subjects to healthy subjects. *p* values, odds ratios (OR), their corresponding 95% confidence intervals (95% CI), and the areas under the ROC curve (AUC) were calculated. Due to the small units, the OR of the raw values are partly very large (e.g., OR = 1596047391). Thus, we presented the ORs for the data as multiplied with 100. All genes and co-variables (gender and age) with a p value<0.00357 (0.05/14: Bonferroni adjustment for multiplicity were further considered in a stepwise multiple logistic regression model. To avoid multicollinearity, a correlation of R>0.6 between two variables in the resulting model was not tolerated. The same approach was chosen for the analysis of AD *vs.* healthy subjects (since no significant result was found in the logistic regression, no multiple model was calculated). Correlation analyses were performed between genes and the factors gender, age, CDR, MMSE, UPDRS and Hamilton depression scale scores. *p* values<0.008 were considered significant (Bonferroni adjusted).

To investigate the validity of the gene measurements, Pearson correlation coefficients were calculated for the values of the first and the second recruitment (only for PD and healthy subjects). Intraclass correlation coefficients were calculated for the values of the first and second recruitments (for all groups). *p* values<0.0042 were considered significant (Bonferroni adjusted). All computations were completed using the statistical computing environment R version 2.8 (http://www.r-project.org/, Department of Statistics and Mathematics of the WU Vienna, Austria) and SAS 9.1 (SAS Institute Inc., Cary, NC, USA).

## Example 1: Evaluation of the stability of blood reference genes

The relative quantification of expression levels is based on the expression levels of target genes *vs*. one or more reference, i.e., reference or control, genes. The normalization procedure is mandatory in quantitative RT-PCR (qRT-PCR) studies and the reason for the choice of the most stably expressed reference genes is to avoid misinterpretation and low reproducibility of the final results.

We decided to determine the expression stability of five widely used reference genes, in particular, ACTB, GAPDH, ALAS1, PPIA and 60S RPL13A, in human leukocyte samples from PD and healthy age-matched controls, randomly divided between males and females.

RNA from blood samples of patients and controls was extracted and reverse transcribed, and expression was determined by quantitative Real-Time RT-PCR, as described in Materials and Methods.

The expression of the selected control genes in samples was analyzed with two widely used Visual Basic for Applications (VBA) applets, i.e., geNorm, providing a measure of gene expression stability and the mean pairwise variation etween an individual gene and all other tested control genes (Vandesompele *et al.,* 2002); and NormFinder, focusing on finding the gene with the least intra- and inter-group expression variation (Andersen *et al.,* 2004).

The geNorm applet was used to measure the average overall expression stability measure (M) value of remaining reference genes as each successive lowest ranking (least stable) gene is eliminated in stepwise fashion, starting with RPL13A, and the stability of the remaining genes is recalculated. GeNorm classified ACTB and ALAS1 as the best two controls of the group, with GAPDH ranking third (**Table 3**). The stability value determined by the NormFinder software attempts to minimize estimated intra- and inter-group variation, using control *vs*. PD status as the independent grouping variable. Lower values indicate greater stability. The best position in the stability ranking produced by NormFinder was occupied by ACTB, followed by GAPDH and ALAS1 (**Table 3**). As it is recommended to use the three most stable internal control genes for calculation of an RT-PCR normalization factor, the three reference genes GAPDH, ACTB and ALAS1 were selected for optimal normalization (Vandesompele *et al.,* 2002). The relative gene expression level was calculated by dividing the raw expression level of the gene of interest by the geometric mean of the expression levels of three reference genes.

**Table 3:** Stability ranking of the candidate reference genes

| Software | Ranking | | | | |
|---|---|---|---|---|---|
|  | 1st | 2nd | 3rd | 4th | 5th |
| geNorm (Average M value) | ACTB and ALAS 1* (0.457) | | GAPDH (0.536) | PPIA (0.69) | RPL13A (0.922) |
| NormFinder (Average Stability Value) | ACTB (0.061) | GAPDH (0.133) | ALAS 1 (0.229) | RPL13A (0.265) | PPIA (0.275) |
| * 1st and 2nd positions cannot be further ranked by geNorm. | | | | | |

## Example 2: Identifying a PD risk marker panel in peripheral blood

In order to identify a PD risk marker panel in peripheral blood with high probability to detect early PD, we have focused on non-medicated *de novo* PD patients to track for gene changes at very early stages of the disease and to ascertain no confounding bias that could arise from medication.

The transcriptional expression level of eight genes, in particular, ALDH1A1, PSMC4, SKP1A, HSPA8, CSK, HIP2 and EGLN1, which have previously been found to be altered in substantia nigra tissue from sporadic PD patients (Grünblatt *et al.,* 2004); and CLTB, elected from the transcriptomic PD blood analysis of Scherzer *et al.* (2007), were assessed in blood samples from 38 individuals with *de-novo* PD, and 64 healthy age-matched controls without neurological dysfunction (**Table 1**).

RNA from blood samples obtained from each one of those individuals was extracted and reverse transcribed, and expression level of each one of said genes was determined by quantitative Real-Time RT-PCR, as described in Materials and Methods.

The relative gene expression was normalized to the geometric mean of the three most stable internal control reference genes GAPDH, ACTB and ALAS1. A stepwise multivariate logistic regression analysis of the natural logarithms of the relative gene expression for all eight genes, with acceptance threshold of $p<0.05$, was carried out as described in Materials and Methods and identified six of these genes, in particular ALDH1A1, PSMC4, HSPA8, SKP1A, HIP2 and EGLN1, as optimal predictors of PD risk.

As shown in **Table 4,** in which negative regression coefficients (B) indicate an inverse relationship between transcript expression and risk for PD, the expression of the genes ALDH1A1, PSMC4, SKP1A and EGLN1 significantly decreased the risk for PD diagnosis, with odds ratio (OR) values of 0.80, 0.74, 0.77 and 0.83, respectively, whereas the expression of HSPA8 and HIP2, having positive regression coefficients, significantly increased the risk for PD diagnosis with OR values of 1.54 and 1.27, respectively.

**Table 4:** Variables in the predicted probability equation

| | **B** | ***p* value** | **OR** | **95% CI (corresponding to OR)** |
|---|---|---|---|---|
| L_ALDH1A1 | -0.2201 | 0.011 | 0.802 | 0.677-0.951 |
| L_HSPA8 | 0.4353 | 0.002 | 1.545 | 1.176-2.032 |
| L_PSMC4 | -0.3059 | 0.009 | 0.736 | 0.586-0.926 |
| L_SKP1A | -0.2608 | 0.026 | 0.770 | 0.612-0.970 |
| L_HIP2/UBE2K | 0.2424 | 0.030 | 1.274 | 1.023-1.587 |
| L_EGLN1 | -0.1899 | 0.035 | 0.827 | 0.693-0.987 |
| L_: Natural logarithm of the relative expression level multiplied by 10 to avoid skewed OR values; CI: Confidence Interval; B: regression coefficient; OR: odds ratio; | | | | |

The predicted probability for PD (p(PD)) in a tested individual was calculated using the regression coefficient values B obtained from the logistic regression model via the following equation (A):

$$(\mathbf{A}) \quad p(PD)=e^{N}/(1+e^{N}),$$

wherein $N=-2.0777+ \sum_{i=1-6} (B_i \cdot 10 \cdot \ln(Gene\_exp_i))$, wherein each i in said formula indicates a different gene i out of the following six genes: ALDH1A1, PSMC4, HSPA8, SKP1A, HIP2 and EGLN1; $B_i$ is the regression coefficient value of said gene i as listed in **Table 4;** and $Gene\_exp_i$ is the relative expression level of said gene i in said individual.

A ROC curve was built from the probability model to calculate the relationship between sensitivity and specificity for the *de-novo* PD group *vs*. healthy controls, and thus evaluate the diagnostic performance of the identified gene cluster (**Fig**. **1**). At the cut-off point of approximately 0.5 we were able to distinguish between non-medicated early PD individuals and healthy controls with sensitivity and specificity values of 87% and 92% respectively (noted with arrows). The area under the curve (AUC) was 0.96. AUC is equal to the probability that a classifier will rank a randomly chosen positive instance higher than a randomly chosen negative one.

Demographic analysis revealed no significant difference between the *de novo* PD group and the control group in age (*t* test, $p=0.075$). Moreover, age did not influence the predicted probability for PD (Pearson correlation, r=0.009, p=0.919) and thus it can be assumed that age was not a predictor of PD risk. The proportion of males was significantly higher in the *de novo* PD group (Mann-Whitney non-parametric test, p=0.016); however, the impact of the gender factor on the predicted probability for PD was not significant (*t* test, $p=0.123$).

**Example 3: Characterizing partial risk marker panels**

We next tested the ability of partial risk marker panels, including genes selected from the full six-genes risk marker panel, established and described in Example 2, to differentiate between PD *de novo* patients and healthy controls. A stepwise multivariate logistic regression analysis was conducted as described in Materials and Methods, and stopped after finding three, four, or five genes of the full six genes panel. A ROC curve was used to calculate the relationship between sensitivity and specificity for the *de-novo* PD group *vs.* healthy controls for each of the partial risk marker panels, and thus evaluate the diagnostic performance of the identified gene clusters. The results are presented in **Table 5A** and regression coefficient (B) values for the predicted probability equation for each partial panel are given in **Table 5B.**

The predicted probability for PD (p(PD)) for each one of these partial risk panels can be calculated using the regression coefficient values (B) and the constant value obtained from the logistic regression model and presented in **Table 5B,** via the following equation (B):

$$\textbf{(B)} \quad p(PD) = e^N/(1+e^N),$$

wherein $N = \text{constant}(GP) + \sum_{i=1-n} (B_i \cdot 10 \cdot \ln(\text{Gene\_exp}_i))$; wherein GP represents the 3-, 4-, or 5-gene panel, and constant(GP) is the constant determined for each one of these gene panels as listed in **Table 5B;** n is 3, 4 or 5, respectively; each i in said formula indicates a different gene i out of the following five genes: ALDH1A1, PSMC4, HSPA8, SKP1A and HIP2; $B_i$ is the regression coefficient value of said gene i in the 3-, 4-, or 5-gene panel as listed in **Table 5B;** and $\text{Gene\_exp}_i$ is the relative expression level of said gene i in said individual. A predicted probability higher than 0.5 is indicative of a positive diagnosis of PD.

**Table 5A:** Sensitivity and specificity of partial risk marker panels

| Panel type | Genes included in the partial panel | Specificity | Sensitivity |
|---|---|---|---|
| 3 genes | ALDH1A1, PSMC4, HSPA8 | 89.06 | 78.95 |
| 4 genes | ALDH1A1, PSMC4, HSPA8, SKP1A | 87.50 | 84.21 |
| 5 genes | ALDH1A1, PSMC4, HSPA8, SKP1A, HIP2 | 90.63 | 86.84 |
| 6 genes | ALDH1A1, PSMC4, HSPA8, SKP1A, HIP2, EGLN1 | 92.19 | 86.84 |

**Table 5B:** Variables for the predicted probability equation for partial panels

| | Regression coefficient (B) | | |
|---|---|---|---|
| | **3-gene panel** | **4-gene panel** | **5-gene panel** |
| L_ALDH1A1 | -0.2393 | -0.1782 | -0.1905 |
| L_PSMC4 | -0.3219 | -0.2838 | -0.3543 |
| L_HSPA8 | 0.4354 | 0.4384 | 0.4110 |
| L_ SKP1A | | -0.1818 | -0.2361 |
| L_HIP2/UBE2K | | | 0.2042 |
| Constant | 0.1760 | -0.8176 | -0.4751 |
| L_: Natural logarithm of the relative expression level multiplied by 10 to avoid skewed OR values. | | | |

## Example 4: Validations of the risk marker panel

To corroborate the above findings by an independent method, a cross-validation of blood expression levels from 100 randomly allocated independent test sets was conducted in which 50% of the *de novo* PD patients and 50% of the healthy age-matched controls were used as a "training set" to generate an independent multivariate discriminative model based on the six-gene risk marker panels found by the logistic regression analysis as described above. The resulting model was applied to the remaining 50% *de novo* PD subjects and controls correctly classifying 78.05% ± 10.66% of PD cases (sensitivity) and 87.23 ± 7.00% (specificity) of controls on average (using a threshold probability of 0.5), basically confirming the findings initially obtained (see also **Fig**. 2).

As a more rigorous validation of the diagnostic value of the PD risk marker panel, the logistic regression model based on the six-gene risk marker panel obtained from the *de novo* PD and healthy control samples was applied to a separate PD cohort consisting of 40 patients under medication at early and advanced disease stages.

Expression levels for the six risk marker panel genes and the three reference genes were determined for each individual, and relative expression levels were calculated as described in Materials and Methods. The predicted probability was calculated for each individual according to the predicted probability equation (A) in Example 2, and each individual was classified as PD or non-PD based on the result.

The risk marker panel displayed a high sensitivity (82.5%) positively classifying 33 out of 40 patients as PD. Additionally, in a sample of 10 patients with the most common neurodegenerative disorder, AD, the risk marker panel displayed 100% specificity correctly classifying all AD individuals as non-PD.

In order to examine the partial risk marker panels, a similar experiment was conducted on the same group of patients, calculating the predicted probability and classifying patients as PD or non-PD based on equation (B) in Example 3 and the three-, four- or five-gene risk marker panels. As found, the partial panels displayed a specificity of 100%, identifying all 10 AD patients as non-PD, and a high degree of sensitivity, between 70 and 85%, positively identifying between 28 and 34 of the 40 PD patients as PD.

### Example 5: Correlation analyses in controls and PD patients

A correlation analysis in the control group subjects between the expression levels of the eight genes measured in Example 2 (**Table 6A**) revealed a gene cluster composed of SKP1A, HIP2, ALDH1A1 and PSMC4, all part of the six-gene risk panel, that showed a significant association in their expression levels. Notably, SKP1A significantly correlated with 6 out of the 7 other transcripts, HIP2, ALDH1A1, PSMC4, HSPA8, EGLN1 and CLTB. In sharp contrast, both the gene cluster and the SKP1A gene correlations were disrupted in the PD *de novo* group (**Table 6B**), pointing to a coordinated expression pattern of selected genes in blood from healthy individuals.

**Table 6A:** Correlations between relative gene expression levels in controls

|  | HIP2 | ALDH1A1 | PSMC4 | HSPA8 | CSK | EGLN1 | CLTB |
|---|---|---|---|---|---|---|---|
| **SKP1A** | R=0.440**<br>P<0.001 | R=0.592**<br>P<0.001 | R=0.466**<br>P<0.001 | R=0.288*<br>P=0.021 | R=-0.217<br>P=0.196 | R=0.283*<br>P=0.023 | R=0.255*<br>P=0.044 |
| **HIP2** | - | R=0.373**<br>P<0.001 | R=0.531**<br>P<0.001 | R=0.227<br>P=0.073 | R=-0.265<br>P=0.112 | R=0.285*<br>P=0.024 | R=0.085<br>P=0.506 |
| **ALDH1A1** |  | - | R=0.329*<br>P=0.008 | R=0.187<br>P=0.142 | R=-0.060<br>P=0.724 | R=0.241<br>P=0.057 | R=0.030<br>P=0.817 |
| **PSMC4** |  |  | - | R=0.367**<br>P=0.003 | R=0.052<br>P=0.761 | R=0.229<br>P=0.071 | R=0.269*<br>P=0.033 |
| **HSPA8** |  |  |  | - | R=0.237<br>P=0.158 | R=0.185<br>P=0.144 | R=0.138<br>P=0.282 |
| **CSK** |  |  |  |  | - | R=0.112<br>P=0.508 | R=0.296<br>P=0.075 |
| **EGLN1** |  |  |  |  |  | - | * R=0.283<br>P=0.030 |

**Table 6B:** Correlations between relative gene expression levels in *de novo* patients

|  | HIP2 | ALDH1A1 | PSMC4 | HSPA8 | CSK | EGLN1 | CLTB |
|---|---|---|---|---|---|---|---|
| **SKP1A** | R=0.115<br>P=0.491 | R=0.128<br>P=0.444 | R=-0.033<br>P=0.845 | R=0.039<br>P=0.816 | R=0.011<br>P=0.951 | R=-0.395*<br>P=0.014 | R=0.201<br>P=0.227 |
| **HIP2** | - | R=0.101<br>P=0.546 | R=0.480**<br>P=0.002 | R=0.496**<br>P=0.002 | R=0.377*<br>P=0.023 | R=0.317<br>P=0.053 | R=0.409*<br>P=0.011 |
| **ALDH1A1** |  | - | R=0.068<br>P=0.684 | R=0.065<br>P=0.699 | R=-0.106<br>P=0.538 | R=-0.255<br>P=0.122 | R=0.210<br>P=0.205 |
| **PSMC4** |  |  | - | R=0.550**<br>P<0.001 | R=0.250<br>P=0.141 | R=0.441**<br>P=0.006 | R=0.321*<br>P=0.049 |

(continued)

| | HIP2 | ALDH1A1 | PSMC4 | HSPA8 | CSK | EGLN1 | CLTB |
|---|---|---|---|---|---|---|---|
| HSPA8 | | | | - | R=0.371*<br>P=0.026 | R=0.292<br>P=0.075 | R=0.334*<br>P=0.041 |
| CSK | | | | | - | R=0.088<br>P=0.610 | R=0.389*<br>P=0.019 |
| EGLN1 | | | | | | - | R=0.154<br>P=0.357 |
| * *p*<0.05; ** *p*<0.01; * R=Pearson correlations coefficient. | | | | | | | |

**Example 6: Differential gene expression of the genes used to build the risk marker** panel

Next, we summarized the individual mRNA relative expression levels of the eight genes used to build the risk marker panel in the five cohorts of subjects. Blood samples were taken from 38 PD *de novo* patients (DN), 24 early PD patients within the first year of medication, H&Y 1-2 (Med. Early), 16 medicated PD patients with advanced disease, H&Y 2.5-4 (Med. Adv.), 10 patients with AD, and 64 healthy age-matched healthy controls without personal or family history of neurodegenerative diseases (Control). Relative expression levels were calculated by dividing the raw quantities for each sample by the geometric mean of the raw quantities of the reference genes ACTB, ALAS1 and GAPDH. The significance was calculated by One-way ANOVA, after post-Hoc Tukey correction, and the results are shown in **Figs. 3A-3H.** Differential expression of each gene revealed significant transcripts level reductions in ALDH1A1 (**3A**), SKP1A (**3C**) and PSMC4 (**3B**), and a significant elevation in HSPA8 (**3D**) among the three PD groups compared to healthy controls.

ALDH1A1, PSMC4 and HSPA8 expression levels did not differ between the three PD cohorts (ALDH1A1: 47.9±3.1, 57.6±5.0 and 58.1±6.5% of control; PSMC4: 68.5±3.1, 67.1±4.2 and 68.9±5.8 % of control; HSPA8: 150.0±13.0, 153.7±16.4 and 153.3±14.4 % of control, respective to *de novo* PD, early medicated PD and advanced stage PD). However, the decline in SKP1A mRNA expression in both early and late medicated groups was significantly more pronounced (40.2±3.1 and 40.9±4.6 % of control, respectively) compared to *de novo* PD cohort (55.1±3.3 % of control). EGLN1 transcript levels decreased only in the early diagnosed, non-medicated group (78.8±6.6 % of control, **3E**).

On the other hand, no significant gene alterations were encountered in CSK (**3F**) and HIP2 (**3G**) in newly diagnosed non-medicated PD compared to control, whereas a clear increase was seen in medicated individuals at early or advanced PD stages. No significant changes in CLTB expression was seen in any of the three PD groups (**3H**). All transcripts levels in the AD group did not significantly differ from the control, except CLTB (CSK was not determined in the AD group).

**Example 7: Additional biomarkers for predicting Parkinson's disease**

A separate set of independent experiments was carried out to find additional biomarkers useful for diagnosing PD in blood. In these experiments, the starting set of genes the transcriptional expression levels of which were measured was partially overlapping with the set of genes used in Examples 1-6, and included the 12 following genes: HSPA8; PSMA2; PSMA3; PSMA5; HS3ST2; SLC31A2; LAMB2; ALDH1A1; HIP2; CSK; CNR2; and HIST1H3E. The reference genes R18S, ACTB, ALAS1, GAPDH, RPL13A and PPIA were used as internal controls. Comparison was made between controls and PD patients, but unlike the procedure described in Examples 1-6, without specifically comparing to unmedicated *de novo* patients, which constituted about 10% of the PD-patients in these experiments.

Univariate logistic regression was conducted for the aforesaid genes and co-variables (gender, age, CDR, MMSE, UPDRS, and Hamilton depression scale scores) for PD *vs.* control subjects. The *p* values, ORs (given for one hundredth of the measurements) and AUCs for the analysis of PD *vs.* controls are presented in **Table 7.** As shown, the genes PSMA2, PSMA3, SLC31A2, LAMB2, ALDH1A1 and HIP2 significantly increased the risk for PD diagnosis, while HIST1H3E significantly decreased the risk for PD diagnosis. Increased UPDRS scores were significantly associated with increased risk of PD diagnosis.

**Table 7:** Logistic regression: PD patients *vs.* controls

| Gene | *p* value | Adjusted *p* value | OR (95% CI) | AUC |
|---|---|---|---|---|
| HSPA8 | 0.014 | 0.24 | 1.04 (1.01-1.07) | 0.674 |

(continued)

| Gene | *p* value | Adjusted *p* value | OR (95% CI) | AUC |
|---|---|---|---|---|
| PSMA5 | 0.475 | 1 | 1.01 (0.98-1.03) | 0.614 |
| PSMA2 | 2.68E-05 | 0.00037 | 1.24 (1.15-1.33) | 0.872 |
| PSMA3 | 0.002 | 0.028 | 1.06 (1.02-1.1) | 0.714 |
| HS3ST2 | 0.505 | 1 | 1.08 (0.86-1.36) | 0.524 |
| SLC31A2 | 2.00E-05 | 0.00028 | 1.12 (1.06-1.18) | 0.847 |
| LAMB2 | 0.00039 | 0.0055 | 2.54 (1.52-4.26) | 0.825 |
| ALDH1A1 | 5.00E-05 | 0.0007 | 1.07 (1.03-1.1) | 0.758 |
| HIP2 | 0.00014 | 0.0020 | 1.05 (1.03-1.08) | 0.743 |
| CSK | 0.004 | 0.056 | 1.03 (1.01-1.06) | 0.742 |
| CNR2 | 0.231 | 1 | 0.99 (0.97-1.01) | 0.546 |
| H1ST1H3E | 0.001 | 0.014 | 0.97 (0.96-0.99) | 0.72 |
| Gender | 0.013 | 0.182 | 2.76 (1.24-6.14) | 0.624 |
| Age | 0.002 | 0.028 | 0.93 (0.89-0.97) | 0.666 |
| For all parameters calculated in the regression, the OR refers to the higher value of the parameter; Adjusted *p* value: the Bonferroni corrected *p* value, significance was set at p<0.05; OR: odds ratio; CI: confidence interval; AUC: area under the ROC curve; Gender: females=0, males=1. | | | | |

All genes or risk factors with *p* values<0.00357 (genes, gender and age) were further selected for multiple analysis. The model identified the following significant genes: PSMA2 (*p*=0.0002, OR=1.15 95% CI 1.07-1.24), LAMB2 (*p*=0.0078, OR=2.26 95% CI 1.24-4.14), ALDH1A1 (*p*=0.016, OR=1.05 95% CI 1.01-1.1), and HISTIH3E (*p*=0.03, OR=0.975 95% CI 0.953-0.998) for PD *vs.* control. The ROC curve was built with these four significant gene biomarkers (**Fig. 4;** max rescaled R2 (correlation coefficient)=0.62; AUC=0.92). Using these four biomarkers for PD diagnosis, sensitivity and specificity of more than 80% (e.g. 91.5% sensitivity and 82% specificity, noted with arrows), were achieved. It should be noted that there are more than 50 possible models out of the set of univariate significant genes with an AUC larger than 0.87 and excluded correlations>0.6 between the variables in the model. All these models have similar sensitivity and specificity of more than 80%.

Correlation analysis between the genes and the factors age, gender, MMSE, CDR, Hamilton depression scale and UPDRS scores resulted in some significant correlations between the four genes PSMA2, LAMB2, ALDH1A1 and HIST1H3E, and the parameters age, MMSE and UPDRS scores (**Table 8**). Increased age and UPDRS scores significantly correlated with the PSMA2 gene expression profile (decreased expression and increased expression, respectively). Nominal significance was found between MMSE scores and LAMB2 (increased expression with decreased score), age and ALDH1A1 (decreased expression with increased age), and UPDRS score and HIST1H3E (decreased expression with increased score).

**Table 8:** Correlations between genes and factors

| Gene | Factor | Correlation coefficient | *P* value | Adjusted *p* value |
|---|---|---|---|---|
| PSMA2 | Age | -0.274 | **0.001** | **0.006** |
| | MMSE | -0.149 | 0.08 | 0.48 |
| | UPDRS score | 0.331 | **<0.001** | **<0.001** |
| | CDR score | -0.102 | 0.231 | 1 |
| | Hamilton depression score | -0.186 | 0.059 | 0.354 |
| | Gender | 0.035 | 0.258 | 1 |

(continued)

| Gene | Factor | Correlation coefficient | P value | Adjusted p value |
|---|---|---|---|---|
| LAMB2 | Age | -0.077 | 0.37 | 1 |
| | MMSE | -0.235 | **0.005** | **0.03** |
| | UPDRS score | 0.101 | 0.239 | 1 |
| | CDR score | 0.157 | 0.065 | 0.36 |
| | Hamilton depression score | 0.121 | 0.22 | 1 |
| | Gender | 0.167 | 0.558 | 1 |
| ALDH1A1 | Age | -0.238 | **0.005** | **0.03** |
| | MMSE | 0.059 | 0.489 | 1 |
| | UPDRS score | 0.12 | 0.16 | 0.96 |
| | CDR score | -0.109 | 0.203 | 1 |
| | Hamilton depression score | -0.045 | 0.647 | 1 |
| | Gender | 0.159 | 0.066 | 0.396 |
| HIST1 H3E | Age | 0.15 | 0.084 | 0.504 |
| | MMSE | 0.038 | 0.666 | 1 |
| | UPDRS score | -0.189 | 0.029 | 0.175 |
| | CDR score | 0.176 | 0.042 | 0.252 |
| | Hamilton depression score | 0.012 | 0.905 | 1 |
| | Gender | -0.005 | 0.53 | 1 |

Adjusted p value: Bonferroni corrected p value; significance was set at p<0.05; Significant results are indicated in bold.

**Tables 9A-9B** show the correlation between the various genes whose expression level was measured in both PD patients and controls, and the numbers represent the correlation coefficient (R) according to the regression. Only Rs>0.5 were considered significant and marked in bold.

**Table 9A:** Correlations coefficient (R) between the genes (part A)

| | HSPA8 | SKP1A | PSMA5 | PSMA2 | PSMA3 | PSMC4 | HS3ST2 | EGLN1 |
|---|---|---|---|---|---|---|---|---|
| **HSPA8** | 1 | **0.58** | **0.65** | 0.34 | 0.38 | **0.54** | 0.25 | 0.38 |
| **SKP1A** | **0.58** | 1 | **0.63** | 0.44 | 0.56 | **0.66** | 0.44 | 0.4 |
| **PSMA5** | **0.65** | **0.63** | 1 | 0.28 | 0.3 | 0.48 | 0.48 | 0.46 |
| **PSMA2** | 0.34 | 0.44 | 0.28 | 1 | 0.45 | 0.48 | 0.16 | 0.31 |
| **PSMA3** | 0.38 | **0.56** | 0.3 | 0.45 | 1 | 0.35 | 0.01 | 0.26 |
| **PSMC4** | **0.54** | **0.66** | 0.48 | 0.48 | 0.35 | 1 | 0.22 | 0.36 |
| **HS3ST2** | 0.25 | 0.44 | 0.48 | 0.16 | 0.01 | 0.22 | 1 | 0.19 |
| **EGLN1** | 0.38 | 0.4 | 0.46 | 0.31 | 0.26 | 0.36 | 0.19 | 1 |
| **SLC31A2** | 0.48 | **0.67** | 0.49 | 0.43 | 0.34 | 0.7 | 0.29 | 0.46 |
| **LAMB2** | 0.29 | **0.59** | 0.42 | 0.37 | 0.28 | 0.34 | 0.54 | 0.26 |
| **ALDH1A1** | 0.27 | **0.5** | 0.24 | 0.32 | 0.47 | 0.44 | 0.04 | 0.26 |
| **HIP2** | 0.43 | **0.6** | 0.46 | 0.28 | 0.37 | **0.61** | 0.08 | 0.32 |
| **CSK** | **0.54** | **0.64** | **0.55** | 0.4 | 0.43 | **0.55** | 0.2 | **0.51** |

(continued)

|  | HSPA8 | SKP1A | PSMA5 | PSMA2 | PSMA3 | PSMC4 | HS3ST2 | EGLN1 |
|---|---|---|---|---|---|---|---|---|
| **CNR2** | 0.04 | -0.07 | 0.02 | -0.05 | -0.07 | -0.1 | 0.01 | -0.11 |
| **HIST1H3E** | -0.02 | -0.06 | -0.04 | -0.15 | -0.09 | -0.21 | 0.22 | -0.08 |

**Table 9B:** Correlations coefficient (R) between the genes (part B)

|  | SLC31A2 | LAMB2 | ALDH1A1 | HIP2 | CSK | CNR2 | HIST1H3E |
|---|---|---|---|---|---|---|---|
| **HSPA8** | 0.48 | 0.29 | 0.27 | 0.43 | **0.54** | 0.04 | -0.02 |
| **SKP1A** | **0.67** | **0.59** | **0.5** | **0.6** | **0.64** | -0.07 | -0.06 |
| **PSMA5** | 0.49 | 0.42 | 0.24 | 0.46 | **0.55** | 0.02 | -0.04 |
| **PSMA2** | 0.43 | 0.37 | 0.32 | 0.28 | 0.4 | -0.05 | -0.15 |
| **PSMA3** | 0.34 | 0.28 | 0.47 | 0.37 | 0.43 | -0.07 | -0.09 |
| **PSMC4** | 0.7 | 0.34 | 0.44 | **0.61** | **0.55** | -0.1 | -0.21 |
| **HS3ST2** | 0.29 | **0.54** | 0.04 | 0.08 | 0.2 | 0.01 | 0.22 |
| **EGLN1** | 0.46 | 0.26 | 0.26 | 0.32 | **0.51** | -0.11 | -0.08 |
| **SLC31A2** | 1 | 0.46 | **0.52** | **0.59** | **0.63** | -0.02 | -0.16 |
| **LAMB2** | 0.46 | 1 | 0.15 | 0.22 | 0.37 | -0.04 | -0.07 |
| **ALDH1A1** | **0.52** | 0.15 | 1 | **0.52** | 0.27 | 0 | -0.11 |
| **HIP2** | **0.59** | 0.22 | **0.52** | 1 | **0.6** | -0.01 | -0.17 |
| **CSK** | **0.63** | 0.37 | 0.27 | **0.6** | 1 | -0.03 | -0.08 |
| **CNR2** | -0.02 | -0.04 | 0 | -0.01 | -0.03 | 1 | 0.29 |
| **HIST1H3E** | -0.16 | -0.07 | -0.11 | -0.17 | -0.08 | 0.29 | 1 |

**Example 8: Validation of the risk marker panel**

Validation analysis was conducted using 67 subjects (37 female and 30 male) with a mean age of 65.67 ± 10 years, who were reinvestigated in a second recruitment of PD, AD and healthy control subjects. Correlation analyses were calculated to obtain a measure for the reproducibility of the gene measurements conducted for the first recruitment. Some genes showed high reproducibility **(Table 10),** e.g., three genes from the multiple models for PD *vs.* healthy (PSMA2, LAMB2 and ALDH1A1). Two genes showed only nominal significance for reproducibility (PSMA3 and CNR2), while two genes (HS3ST2 and HIST1H3E) had low reproducibility rates. For the second recruitment, a multiple model including the variables PSMA2, LAMB2, ALDH1A1 and HISTIH3E was used to calculate the AUC (max rescaled $R^2$=0.66, AUC=0.93), which resulted in sensitivity and specificity of more than 80% (data not shown).

**Table 10:** Correlation between first and second recruitment

| Gene | Correlation Coefficient | P value | Adjusted p value |
|---|---|---|---|
| HSPA8 | 0.605 | **1.02E-03** | **0.0122** |
| PSMA5 | 0.479 | **0.00021** | **0.0025** |
| PSMA2 | 0.566 | **6.68E-03** | **0.0801** |
| PSMA3 | 0.294 | 0.029 | 0.348 |
| HS3ST2 | 0.073 | 0.596 | 1 |
| SLC31A2 | 0.57 | **5.50E-06** | **6.6E-05** |
| LAMB2 | 0.58 | **3.40E-06** | **4.08E-05** |

(continued)

| Gene | Correlation Coefficient | P value | Adjusted p value |
|---|---|---|---|
| ALDH1A1 | 0.447 | 0.001 | **0.012** |
| HIP2 | 0.744 | **8.00E-11** | **9.6E-10** |
| CSK | 0.478 | **0.00022** | **0.0026** |
| CNR2 | 0.277 | 0.043 | 0.516 |
| HIST1H3E | 0.252 | 0.066 | 0.792 |

Adjusted *p* value: the Bonferroni corrected *p* value; significance was set at $p<0.05$; Significant results are indicated in bold.

## REFERENCES

Andersen C.L., Jensen J.L., Orntoft T.F., Normalization of real-time quantitative reverse transcription-PCR data: a model-based variance estimation approach to identify genes suited for normalization, applied to bladder and colon cancer data sets. Cancer research, 2004, 64(15), 5245-5250

Berg D. Biomarkers for the early detection of Parkinson's and Alzheimer's disease. Neurodegener Dis., 2008, 5(3-4), 133-136

Berg L., Clinical Dementia Rating (CDR). Psychopharmacol Bull, 1988, 24, 637-639

Braak H., Muller C.M., Rub U., Ackermann H., Bratzke H., de Vos R.A., Del Tredici K., Pathology associated with sporadic Parkinson's disease - where does it end? Journal of neural transmission, 2006, 70, 89-97

Brooks D.J., Technology insight: imaging neurodegeneration in Parkinson's disease. Nat Clin Pract Neurol., 2008, 4(5), 267-277

Ciechanover A., Brundin P., The ubiquitin proteasome system in neurodegenerative diseases: sometimes the chicken, sometimes the egg. Neuron., 2003, 40, 427-446

Dauer W., Przedborski S., Parkinson's disease: mechanisms and models. Neuron., 2003, 39(6), 889-909

Davis J.W., Grandinetti A., Waslien C.I., Ross G.W., White L.R., Morens D.M., Observations on serum uric acid levels and the risk of idiopathic Parkinson's disease. American journal of epidemiology, 1996, 144(5), 480-484

Dawson T.M., Dawson V.L., Molecular pathways of neurodegeneration in Parkinson's disease. Science, 2003, 302, 819-822

De Pril R., Fischer D.F., Roos R.A., van Leeuwen F.W., Ubiquitin-conjugating enzyme E2-25K increases aggregate formation and cell death in polyglutamine diseases. Mol Cell Neurosci., 2007, 34, 10-19

Eckert T., Tang C., Eidelberg D., Assessment of the progression of Parkinson's disease: a metabolic network approach. Lancet Neurol., 2007, 6(10), 926-932

Eller M., Williams D.R., Biological fluid biomarkers in neurodegenerative parkinsonism. Nature reviews, 2009, 5(10), 561-570

Fahn S., Elton R., UPDRS Development Committee. Unified Parkinson's disease rating scale. In: Fahn S., Marsden C.D., Goldstein M., editors, Recent Developments in Parkinson's Disease. New York: Macmillan, 1987, 153-167

Fearnley J.M., Lees A.J, Ageing and Parkinson's disease: substantia nigra regional selectivity. Brain, 1991, 114 (5), 2283-2301

Feldman R.M., Correll C.C., Kaplan K.B., Deshaies R.J., A complex of Cdc4p, Skplp, and Cdc53p/oullin catalyzes ubiquitination of the phosphorylated CDK inhibitor Siclp. Cell, 1997, 91(2), 221-230

Fishman-Jacob T., Reznichenko L., Youdim M.B., Mandel S.A., A sporadic Parkinson's disease model via silencing of the ubiquitin-proteasorne/E3-ligase component SKPIA. J Biol Chem., 2009, 284(47), 32835-32845

Grünblatt E., Mandel S., Jacob-Hirach J., Zeligson S., Amariglo N., Rechavi G., Li J., Ravid R., Roggendorf W., Riederer P., Youdim M.B., Gene expression profiling of parkinsonian substantia nigra pars compacta alterations in ubiquitin-proteasome, heat shock protein, iron and oxidative stress regulated proteins, cell adhesion/cellular matrix and vesicle trafficking genes. J Neural Transm, 2004, 111(12), 1543-1573

Grünblatt E., Mandel S., Müller T., Jost W.H., Youdim M.B., Riederer P., Early diagnosis for Parkinson's disease according to whole blood gene profile. XVII WFN World congress on Parkinson's Disease and related disorders, 9-13 December 2007, Amsterdam, The Netherlands.

Grünblatt E., Bartl J., Zehetmayer S., Ringel T.M., Bauer P., Riederer P., Jacob C.P., Gene expression as peripheral biomarkers for sporadic Alzheimer's disease. J Alzheimers Dis, 2009, 16, 627-634

Hamilton M., A rating scale for depression. J Neurol Neurosurg Psychiatry, 1960, 23, 56-62

Hennecke G., Scherzer C.R., RNA biomarkers of Parkinson's disease: developing tools for novel therapies. Biomarkers Med, 2008, 475, 2, 41-53

Hjelle J.J., Petersen D.R., Hepatic aldehyde dehydrogenases and lipid peroxidation. Pharmacol Biochem Behav., 1983, 18 Suppl 1, 155-160

Hoehn M.M., Yahr M.D., Parkinsonism: onset, progression and mortality. Neurology, 1967, 17(5), 427-442

Hong Z., Shi M.. Chung K.A. et al. DJ-1 and alpha-synuclein in human cerebrospinal fluid as biomarkers of Parkinson's disease. Brain, 2010, 133, 713-726

Hughes A.J., Daniel S.E., Kilford L., Lees A.J., Accuracy of clinical diagnosis of idiopathic Parkinson's disease: a clinico-pathological study of 100 cases. Journal of necrology, neurosurgery, and psychiatry, 1992, 55(3), 181-184

Hughes A.J., Daniel S.E., Ben-Shlomo Y., Lees A.J., The accuracy of diagnosis of parkinsonian syndromes in a specialist movement disorder service. Brain, 2002, 125, 861-870

Jankovic J., Rajput A.H. McDermott M.P., Perl D.P., The evolution of diagnosis in early Parkinson disease. Parkinson Study Group. Archives of neurology, 2000, 57(3), 369-372

Maxdh G., Vallee B.L., Human class I alcohol dehydrogenases catalyze the interconversion of alcohols and aldehydes in the metabolism of dopamine. Biochemistry, 1986, 25, 7279-7282

McKhann G., Drachman D., Folstein M., Katzman R., Price D., Stadlan E.M., Clinical diagnosis of Alzheimer's disease: report of the NTNCDS-ADRDA Work Group under the auspices of Department of Health and Human Services Task Force on Alzheimer's disease. Neurology, 1984, 34, 939-944

Mollenhauer B., Cullen V., Kahn I., Krastins B., Outeiro T.F., Pepivani I., Ng J., Schulz-Schaeffer W., Kretzschmar H.A., McLean PJ., Tronkwalder C.. Sarracino D.A., Vonsattel J.P., Locascio JJ., El-Agnaf O.M., Schlossmacher M.G., Direct quantification of CSF alpha-synuclein by ELISA and first cross-sectional study in patients with neurodegeneration. Experimental neurology, 2008, 213(2), 315-325

O'Connor D.W., Pollitt P.A., Hyde J.B., Fellows J.L., Miller N.D., Brook C.P., Reiss B.B., The reliability and validity of the minimental state in a British community survoy. J Psychiatr Res, 1989, 23, 87-96

Scherzer C.R., Eklund A.C., Morse L.J., Liao Z., Locascio J.J., Fefer D., Schwarzschild M.A., Schlossmacher M.G., Hauser M.A., Vance J.M., Sudarsky L.R., Standaert D.G., Growdon J.H., Jensen R.V., Gullans S.R., Molecular markers of early Parkinson's disease based on gene expression in blood. Proceedings of the National Academy of Sciences of the United States of America, 2007, 104(3), 955-960

Schwarzschild M.A., Schwid S.R-, Marek K., Watts A., Lang A.E., Oakes D., Shoulson I., Ascherio A., Hyson C., Gorbold E., Rudolph A., Kicburtz K.., Fahn S., Gauger L., Goetz C., Seibyl J., Forrest M., Ondrasik J., Serum urate as a predictor of clinical and radiographic progression in Parkinson disease. Archives of neurology, 2008, 65(6), 716-723

Sullivan P.F., Fan C., Perou CM., Evaluating the comparability of gene expression in blood and brain. Am J Med Genet B Nouropsychiatr Genet, 2006, 141B(3), 261-268

Tolosa E., Weaning G., Poewe W., The diagnosis of Parkinson's disease. Lancet neurology, 2006, 5(1), 75-86

Vandesompele J., De Preter K., Pattyn F., Poppe B., Van Roy N., De Paepc A., Speleman P., Accurate normalization of real-time quantitative RT-PCR data by geometric averaging of multiple internal control genes. Genome biology, 2002, 3(7), RESEARCH0034

Weisskopf M.G., O'Reilly. E., Chen H., Schwarzschild MA., Ascherio A., Plasma urate and risk of Parkinson's disease. American journal of epidemiology, 2007, 166(5), 561-567

Wouters H., van Gool W.A., Schmand B., Lindeboom R., Revising the ADAS-cog for a more accurate assessment of cognitive impairment. Alzheimer Dis Assoc Disord, 2008, 22(3), 236-244

Zhang J., Sokal I., Peskind E.R., Quinn J.F., Jankovio J., Kenney C., Chung K.A., Millard S.P., Mutt J.G., Montine T.J., CSF multianalyte profile distinguishes Alzheimer and Parkinson diseases. American journal of clinical pathology, 2008, 129(4), 526-529

Zheng B., Liao Z., Locascio J.J., Lesniak K.A., Roderick S,S., et al., PGC-$1\alpha$, a potential therapeutic target for early intervention in Parkinson's disease. Sci Transl Med., 2010, 2(52), 52-73

## Claims

1. A computerized method for diagnosis of Parkinson's disease (PD) in a tested individual comprising analyzing, using a processor, an expression profile representing the normalized expression levels of genes in a blood sample of said individual by subjecting said expression profile to a formula based on a statistical analysis of known expression profiles, said known expression profiles representing the normalized expression level of each one of said genes in PD patients and in control individuals, thereby obtaining a value corresponding to the probability that the tested individual has PD, wherein one of said genes is ALDH1A1 and at least two of said genes are selected from PSMC4, HSPA8, SKP1A, HIP2 and EGLN1.

2. The method of claim 1, wherein

(i) said genes are ALDH1A1, PSMC4 and HSPA8;
(ii) said genes are ALDH1A1, PSMC4, HSPA8 and SKP1A;
(iii) said genes are ALDH1A1, PSMC4, HSPA8, SKP1A and HIP2;
(iv) said genes are ALDH1A1, PSMC4, HSPA8, SKP1A, HIP2 and EGLN1 ;
(v) three of said genes are ALDH1A1, PSMC4 and HSPA8;
(vi) four of said genes are ALDH1A1, PSMC4, HSPA8 and SKP1A;
(vii) five of said genes are ALDH1A1, PSMC4, HSPA8, SKP1A and HIP2; or
(viii) six of said genes are ALDH1A1, PSMC4, HSPA8, SKP1A, HIP2 and EGLN1.

3. The method of claim 1, wherein said genes further include one or more genes selected from ARPP-21, SLC18A2, SRPK2, TMEFF1, TRIM36, ADH5, PSMA3, PSMA2, PSMA5, EIF4EBP2, LGALS9, LOC56920, LRP6, MAN2B1, PARVA, PENK, SELPLG, SPHK1, SRRM2, LAMB2, HIST1 H3E and ZSIG11.

4. The method of claim 3, wherein said one or more genes are PSMA2; LAMB2; HIST1H3E; PSMA2 and LAMB2; PSMA2 and HIST1H3E; LAMB2 and HIST1H3E; or PSMA2, LAMB2 and HIST1 H3E.

5. A method for diagnosis of Parkinson's disease (PD) in a tested individual comprising determining the expression levels of genes in a blood sample of said individual, wherein said genes include ALDH1A1, PSMA2, LAMB2, and HIST1 H3E.

6. A computerized method for diagnosis of Parkinson's disease (PD) in a tested individual comprising analyzing, using a processor, an expression profile representing the normalized expression levels of genes in a blood sample of said individual by subjecting said expression profile to a formula based on a statistical analysis of known expression profiles, said known expression profiles representing the normalized expression level of each one of said genes in PD patients and in control individuals, thereby obtaining a value corresponding to the probability that the tested individual has PD, wherein said genes include ALDH1A1, PSMA2, LAMB2, and HIST1H3E.

7. The method of claim 1 or claim 6, wherein

i. said expression profile is obtained by measuring the expression levels of said genes in said blood sample and normalizing the expression levels measured; or
ii. said value is compared with a predetermined cut-off value, and said value being higher than said cut-off value indicates that the tested individual has PD.

8. The method of claim 5 or claim 6, further comprising determining the expression levels of genes in a blood sample of said individual, wherein said genes include one or more genes selected from PSMC4, HSPA8, SKP1A, HIP2 and EGLN1.

9. The method of claim 5 or claim 6, further comprising determining the expression levels of genes in a blood sample of said individual, wherein said genes include one or more genes selected from PSMC4; HSPA8; SKP1A; PSMC4 and HSPA8; PSMC4 and SKP1A; HSPA8 and SKP1A; and PSMC4, HSPA8 and SKP1A.

10. The method of any one of claims 1 or 6, wherein said statistical analysis is based on a general linear model, such as a logistic regression model.

11. The method of claim 10, wherein said expression profile representing the normalized expression level of each one of said genes in said blood sample is subjected to the formula $P = e^N/(1+e^N)$,
wherein
N represents the weighted sum of the natural logarithms of the normalized expression levels of said genes, with the addition of a constant; and
P corresponds to the probability that the tested individual has PD.

12. The method of claim 11, wherein

i. said expression profile represents the normalized expression levels of the genes ALDH1A1, PSMC4, HSPA8, SKP1A, HIP2 and EGLN1 in a blood sample of said individual, and said expression profile is subjected to the formula:

$$P = e^N / (1+e^N),$$

wherein $N = -2.078 + \sum_{i=1-6} (B_i \cdot 10 \cdot \ln(\text{Gene\_exp}_i))$; each i in said formula indicates a different gene i out of the following six genes: ALDH1A1, PSMC4, HSPA8, SKP1A, HIP2 and EGLN1; $B_i$ is the regression coefficient value of said gene i; $\text{Gene\_exp}_i$ is the relative expression level of said gene i in said individual; B(ALDH1A1) is -0.220; B(PSMC4) is -0.306; B(HSPA8) is 0.435; B(SKP1A) is -0.261; B(HIP2) is 0.242; B(EGLN1) is -0.190; and P corresponds to the probability that the tested individual has PD;

ii. said expression profile represents the normalized expression levels of the genes ALDH1A1, PSMC4, HSPA8, SKP1A and HIP2 in a blood sample of said individual, and said expression profile is subjected to the formula:

$$P = e^N / (1+e^N),$$

wherein $N = -0.475 + \sum_{i=1-5} (B_i \cdot 10 \cdot \ln(\text{Gene\_exp}_i))$; each i in said formula indicates a different gene i out of the following five genes: ALDH1A1, PSMC4, HSPA8, SKP1A and HIP2; $B_i$ is the regression coefficient value of said gene i; $\text{Gene\_exp}_i$ is the relative expression level of said gene i in said individual; B(ALDH1A1) is -0.191; B(PSMC4) is -0.354; B(HSPA8) is 0.411; B(SKP1A) is -0.236; B(HIP2) is 0.204; and P corresponds to the probability that the tested individual has PD;

iii. said expression profile represents the normalized expression levels of the genes ALDH1A1, PSMC4, HSPA8 and SKP1A in a blood sample of said individual, and said expression profile is subjected to the formula:

$$P = e^N / (1+e^N),$$

wherein $N = -0.818 + \sum_{i=1-4} (B_i \cdot 10 \cdot \ln(\text{Gene\_exp}_i))$; each i in said formula indicates a different gene i out of the following four genes: ALDH1A1, PSMC4, HSPA8 and SKP1A; $B_i$ is the regression coefficient value of said gene i; $\text{Gene\_exp}_i$ is the relative expression level of said gene i in said individual; B(ALDH1A1) is -0.178; B(PSMC4) is -0.284; B(HSPA8) is 0.438; B(SKP1A) is -0.182; and P corresponds to the probability that the tested individual has PD; or

iv. said expression profile represents the normalized expression levels of the genes ALDH1A1, PSMC4 and HSPA8 in a blood sample of said individual, and said expression profile is subjected to the formula:

$$P = e^N / (1+e^N),$$

wherein $N = 0.176 + \sum_{i=1-3} (B_i \cdot 10 \cdot \ln(\text{Gene\_exp}_i))$; each i in said formula indicates a different gene i out of the following three genes: ALDH1A1, PSMC4 and HSPA8; $B_i$ is the regression coefficient value of said gene i; $\text{Gene\_exp}_i$ is the relative expression level of said gene i in said individual; B(ALDH1A1) is -0.239; B(PSMC4) is -0.322; B(HSPA8) is 0.435; and P corresponds to the probability that the tested individual has PD.

13. The method of any one of claims 1, 5, and 6, wherein the tested individual has not received PD therapy.

14. A kit for diagnosis of Parkinson's disease (PD) in a tested individual, comprising:

(i) primers and reagents for quantitative real-time PCR amplification and measuring expression levels of genes, wherein the genes are ALDH1A1, PSMA2, LAMB2, and HIST1H3E;
(ii) primers and reagents for quantitative real-time PCR amplification of at least one control gene for normalizing the expression levels measured in (i) to obtain normalized expression levels; and
(iii) instructions for use.

15. The kit of claim 14, further comprising a formula based on a statistical analysis of known expression profiles of the genes measured in PD patients and in control individuals, for applying to the normalized expression levels to obtain a value corresponding to the probability that the tested individual has PD, wherein said instructions include a predetermined cut-off value to which said value is compared.

**Patentansprüche**

1. Computerisiertes Verfahren zur Diagnose der Parkinson-Krankheit (PD, für Englisch ‚Parkinson's disease') in einem getesteten Individuum, umfassend das Analysieren, unter Verwendung eines Verarbeiters, von einem Expressionsprofil, das die normalisierten Expressionsniveaus von Genen in einer Blutprobe des Individuums repräsentiert, indem das Expressionsprofil einer Formel, basierend auf einer statistischen Analyse bekannter Expressionsprofile, unterzogen wird, wobei die bekannten Expressions-profile das normalisierte Expressionsniveau von jedem der Gene in PD-Patienten und in Kontrollindividuen repräsentieren, wodurch ein Wert erhalten wird, der der Wahrscheinlichkeit entspricht, dass das getestete Individuum PD hat, wobei eines von den Genen ALDH1A1 ist und mindestens zwei der Gene aus PSMC4, HSPA8, SKP1A, HIP2 und EGLN1 ausgewählt sind.

2. Verfahren nach Anspruch 1, wobei

   (i) die Gene ALDH1A1, PSMC4 und HSPA8 sind;
   (ii) die Gene ALDH1A1, PSMC4, HSPA8 und SKP1A sind;
   (iii) die Gene ALDH1A1, PSMC4, HSPA8, SKP1A und HIP2 sind;
   (iv) die Gene ALDH1A1, PSMC4, HSPA8, SKP1A, HIP2 und EGLN1 sind;
   (v) drei der Gene ALDH1A1, PSMC4 und HSPA8 sind;
   (vi) vier der Gene ALDH1A1, PSMC4, HSPA8 und SKP1A sind;
   (vii) fünf der Gene ALDH1A1, PSMC4, HSPA8, SKP1A und HIP2 sind; oder
   (viii) sechs der Gene ALDH1A1, PSMC4, HSPA8, SKP1A, HIP2 und EGLN1 sind;

3. Verfahren nach Anspruch 1, wobei die Gene ferner ein oder mehrere Gene umfassen, die aus ARPP-21, SLC18A2, SRPK2, TMEFF1, TRIM36, ADH5, PSMA3, PSMA2, PSMA5, EIF4EBP2, LGALS9, LOC56920, LRP6, MAN2B1, PARVA, PENK, SELPLG, SPHK1, SRRM2, LAMB2, HIST1H3E und ZSIG11 ausgewählt sind.

4. Verfahren nach Anspruch 3, wobei die ein oder mehreren Gene PSMA2; LAMB2; HIST1H3E; PSMA2 und LAMB2; PSMA2 und HIST1H3E; LAMB2 und HIST1 H3E sind.

5. Verfahren zur Diagnose der Parkinson-Krankheit (PD) in einem getesteten Individuum, umfassend das Bestimmen der Expressionsniveaus von Genen in einer Blutprobe des Individuums, wobei die Gene ALDH1A1, PSMA2, LAMB2 und HIST1H3E umfassen.

6. Computerisiertes Verfahren zur Diagnose der Parkinson-Krankheit (PD) in einem getesteten Individuum, umfassend das Analysieren, unter Verwendung eines Verarbeiters, von einem Expressionsprofil, das die normalisierten Expressionsniveaus von Genen in einer Blutprobe des Individuums repräsentiert, indem das Expressionsprofil einer Formel, basierend auf einer statistischen Analyse bekannter Expressionsprofile, unterzogen wird, wobei die bekannten Expressionsprofile das normalisierte Expressionsniveau von jedem der Gene in PD-Patienten und in Kontrollindividuen repräsentieren, wodurch ein Wert erhalten wird, der der Wahrscheinlichkeit entspricht, dass das getestete Individuum PD hat, wobei die Gene ALDH1A1, PSMA2, LAMB2 und HIST1H3E umfassen.

7. Verfahren nach Anspruch 1 oder Anspruch 6, wobei

   i. das Expressionsprofil erhalten wird, indem die Expressionsniveaus der Gene in der Blutprobe gemessen werden und die gemessenen Expressionsniveaus normalisiert werden; oder
   ii. der Wert mit einem vorbestimmten Grenzwert verglichen wird und wobei der Wert, wenn er höher als der Grenzwert ist, anzeigt, dass das getestete Individuum PD hat.

8. Verfahren nach Anspruch 5 oder Anspruch 6, ferner umfassend das Bestimmen der Expressionsniveaus von Genen in einer Blutprobe des Individuums, wobei die Gene ein oder mehrere Gene umfassen, ausgewählt aus PSMC4, HSPA8, SKP1A, HIP2 und EGLN1.

9. Verfahren nach Anspruch 5 oder Anspruch 6, ferner umfassend das Bestimmen der Expressionsniveaus von Genen in einer Blutprobe des Individuums, wobei die Gene ein oder mehrere Gene umfassen, ausgewählt aus PSMC4;

HSPA8; SKP1A; PSMC4 und HSPA8; PSMC4 und SKP1A; HSPA8 und SKP1A; und PSMC4, HSPA8 und SKP1A.

**10.** Verfahren nach einem der Ansprüche 1 oder 6, wobei die statistische Analyse auf einem allgemeinen linearen Modell basiert, wie zum Beispiel einem logistischen Regressionsmodell.

**11.** Verfahren nach Anspruch 10, wobei das Expressionsprofil, das das normalisierte Expressionsniveau von jedem einzelnen der Gene in der Blutprobe repräsentiert, der Formel $P = e^N / (1+e^N)$ unterzogen wird, wobei

N die gewichtete Summe des natürlichen Logarithmus der normalisierten Expressionsniveaus der Gene mit der Addition einer Konstante repräsentiert; und

P der Wahrscheinlichkeit entspricht, dass das getestete Individuum PD hat.

**12.** Verfahren nach Anspruch 11, wobei

i. das Expressionsprofil die normalisierten Expressionsniveaus der Gene ALDH1A1, PSMC4, HSPA8, SKP1A, HIP2 und EGLN1 in einer Blutprobe des Individuums repräsentiert und wobei das Expressionsprofil der folgenden Formel unterzogen wird:

$$P = e^N / (1+e^N),$$

wobei $N = -2{,}078 + \sum_{i=1-6} (B_1 \cdot 10 \cdot \ln(\text{Gene\_exp}_i))$, wobei i in der Formel ein unterschiedliches Gen i aus den folgenden sechs Genen angibt: ALDH1A1, PSMC4, HSPA8, SKP1A, HIP2 und EGLN1; $B_i$ der Regressionskoeffizientenwert des Gens i ist; $\text{Gene\_exp}_i$ das relative Expressionsniveau des Gens i in dem Individuum ist; B(ALDH1A1) -0,220 ist; B(PSMC4) -0,306 ist; B(HSPA8) 0,435 ist; B(SKP1A) -0,261 ist; B(HIP2) 0,242 ist; B(EGLN1) -0,190 ist; und P der Wahrscheinlichkeit entspricht, dass das getestete Individuum PD hat;

ii. das Expressionsprofil die normalisierten Expressionsniveaus der Gene ALDH1A1, PSMC4, HSPA8, SKP1A und HIP2 in einer Blutprobe des Individuums repräsentiert und wobei das Expressionsprofil der folgenden Formel unterzogen wird:

$$P = e^N / (1+e^N),$$

wobei $N = -0{,}475 + \sum_{i=1-5} (B_1 \cdot 10 \cdot \ln(\text{Gene\_exp}_i))$, wobei i in der Formel ein unterschiedliches Gen i aus den folgenden fünf Genen angibt: ALDH1A1, PSMC4, HSPA8, SKP1A und HIP2; $B_i$ der Regressions-koeffizientenwert des Gens i ist; $\text{Gene\_exp}_i$ das relative Expressions-niveau des Gens i in dem Individuum ist; B(ALDH1A1) -0,191 ist; B(PSMC4) -0,354 ist; B(HSPA8) 0,411 ist; B(SKP1A) -0,236 ist; B(HIP2) 0,204 ist; und P der Wahrscheinlichkeit entspricht, dass das getestete Individuum PD hat;

iii. das Expressionsprofil die normalisierten Expressionsniveaus der Gene ALDH1A1, PSMC4, HSPA8 und SKP1A in einer Blutprobe des Individuums repräsentiert und wobei das Expressionsprofil der folgenden Formel unterzogen wird:

$$P = e^N / (1+e^N),$$

wobei $N = -0{,}818 + \sum_{i=1-4} (B_1 \cdot 10 \cdot \ln(\text{Gene\_exp}_i))$; wobei i in der Formel ein unterschiedliches Gen i aus den folgenden vier Genen angibt: ALDH1A1, PSMC4, HSPA8 und SKP1A; $B_i$ der Regressionskoeffi-zientenwert des Gens i ist; $\text{Gene\_exp}_i$ das relative Expressions-niveau des Gens i in dem Individuum ist; B(ALDH1A1) -0,178 ist; B(PSMC4) -0,284 ist; B(HSPA8) 0,438 ist; B(SKP1A) -0,182 ist; und P der Wahrscheinlichkeit entspricht, dass das getestete Individuum PD hat; oder

iv. das Expressionsprofil die normalisierten Expressionsniveaus der Gene ALDH1A1, PSMC4 und HSPA8 in einer Blutprobe des Individuums repräsentiert und wobei das Expressionsprofil der folgenden Formel unterzogen wird:

$$P = e^N / (1+e^N),$$

wobei N = 0,176 + $\sum_{i=1-3}$ (B$_1$·10·ln(Gene_exp$_i$)); wobei i in der Formel ein unterschiedliches Gen i aus den folgenden drei Genen angibt: ALDH1A1, PSMC4 und HSPA8; B$_i$ der Regressionskoeffizientenwert des Gens i ist; Gene_exp$_i$ das relative Expressionsniveau des Gens i in dem Individuum ist; B(ALDH1A1) -0,239 ist; B(PSMC4) -0,322 ist; B(HSPA8) 0,435 ist; und P der Wahrscheinlichkeit entspricht, dass das getestete Individuum PD hat.

13. Verfahren nach einem der Ansprüche 1, 5 und 6, wobei das getestete Individuum keine PD-Therapie erhalten hat.

14. Kit zur Diagnose der Parkinson-Krankheit (PD) in einem getesteten Individuum, umfassend:

(i) Primer und Reagenzien für eine quantitative Real-Time-PCR-Amplifikation und Messen der Expressionsniveaus von Genen, wobei die Gene ALDH1A1, PSMA2, LAMB2 und HIST1 H3E sind;
(ii) Primer und Reagenzien für eine quantitative Real-Time-PCR-Amplifikation von mindestens einem Kontrollgen zum Normalisieren der unter (i) gemessenen Expressionsniveaus, um normalisierte Expressionsniveaus zu erhalten; und
(iii) Anweisungen zur Verwendung.

15. Kit nach Anspruch 14, ferner umfassend eine Formel, basierend auf einer statistischen Analyse bekannter Expressionsprofile von Genen, die in PD-Patienten und Kontrollindividuen gemessen wurden, um sie auf die normalisierten Expressionsniveaus anzuwenden, um einen Wert zu erhalten, der der Wahrscheinlichkeit entspricht, dass das getestete Individuum PD hat, wobei die Anweisungen einen vorbestimmten Grenzwert umfassen, mit dem der Wert verglichen wird.

**Revendications**

1. Procédé informatisé pour le diagnostic de la maladie de Parkinson (PD) chez un individu testé, comprenant l'analyse, en utilisant un processeur, d'un profil d'expression représentant les niveaux d'expression normalisés de gènes dans un échantillon de sang dudit individu en soumettant ledit profil d'expression à une formule basée sur une analyse statistique de profils d'expression connus, lesdits profils d'expression connus représentant le niveau d'expression normalisé de chacun desdits gènes chez des patients atteints de la maladie de Parkinson et chez des individus de contrôle, en obtenant ainsi une valeur correspondant à la probabilité que l'individu testé a la maladie de Parkinson, dans lequel l'un desdits gènes est ALDH1A1 et au moins deux desdits gènes sont choisis à partir de PSMC4, HSPA8, SKP1A, HIP2 et EGLN1.

2. Procédé selon la revendication 1, dans lequel :

(i) lesdits gènes sont ALDH1A1, PSMC4 et HSPA8 ;
(ii) lesdits gènes sont ALDH1A1, PSMC4, HSPA8 et SKP1A ;
(iii) lesdits gènes sont ALDH1A1, PSMC4, HSPA8, SKP1A et HIP2 ;
(iv) lesdits gènes sont ALDH1A1, PSMC4, HSPA8, SKP1A, HIP2 et EGLN1 ;
(v) trois desdits gènes sont ALDH1A1, PSMC4 et HSPA8 ;
(vi) quatre desdits gènes sont ALDH1A1, PSMC4, HSPA8 et SKP1A ;
(vii) cinq desdits gènes sont ALDH1A1, PSMC4, HSPA8, SKP1A et HIP2 ; ou
(viii) six desdits gènes sont ALDH1A1, PSMC4, HSPA8, SKP1A, HIP2 et EGLN1.

3. Procédé selon la revendication 1, dans lequel lesdits gènes comprennent en outre un ou plusieurs gènes choisis à partir de ARPP-21, SLC18A2, SRPK2, TMEFF1, TRIM36, ADH5, PSMA3, PSMA2, PSMA5, EIF4EBP2, LGALS9, LOC56920, LRP6, MAN2B1, PARVA, PENK, SELPLG, SPHK1, SRRM2, LAMB2, HIST1 H3E et ZSIG11.

4. Procédé selon la revendication 3, dans lequel lesdits un ou plusieurs gènes sont PSMA2 ; LAMB2 ; HIST1H3E ; PSMA2 et LAMB2 ; PSMA2 et HIST1 H3E ; LAMB2 et HIST1H3E ; ou PSMA2, LAMB2 et HIST1 H3E.

5. Procédé pour le diagnostic de la maladie de Parkinson (PD) chez un individu testé, comprenant la détermination des niveaux d'expression de gènes dans un échantillon de sang dudit individu, dans lequel lesdits gènes comprennent ALDH1A1, PSMA2, LAMB2, et HIST1 H3E.

6. Procédé informatisé pour le diagnostic de la maladie de Parkinson (PD) chez un individu testé, comprenant l'analyse, en utilisant un processeur, d'un profil d'expression représentant les niveaux d'expression normalisés de gènes dans un échantillon de sang dudit individu en soumettant ledit profil d'expression à une formule basée sur une analyse statistique de profils d'expression connus, lesdits profils d'expression connus représentant le niveau d'expression normalisé de chacun desdits gènes chez des patients atteints de la maladie de Parkinson et chez des individus de contrôle, en obtenant ainsi une valeur correspondant à la probabilité que l'individu testé a la maladie de Parkinson, dans lequel lesdits gènes comprennent ALDH1A1, PSMA2, LAMB2, et HIST1 H3E.

7. Procédé selon la revendication 1 ou la revendication 6, dans lequel

    (i) ledit profil d'expression est obtenu en mesurant les niveaux d'expression desdits gènes dans ledit échantillon de sang et en normalisant les niveaux d'expression mesurés ; ou
    (ii) ladite valeur est comparée avec une valeur de coupure prédéterminée, et lorsque ladite valeur est plus grande que ladite valeur de coupure cela indique que l'individu testé a la maladie de Parkinson.

8. Procédé selon la revendication 5 ou la revendication 6, comprenant en outre la détermination des niveaux d'expression de gènes dans un échantillon de sang dudit individu, dans lequel lesdits gènes comprennent un ou plusieurs gènes choisis à partir de PSMC4, HSPA8, SKP1A, HIP2 et EGLN1.

9. Procédé selon la revendication 5 ou la revendication 6, comprenant en outre la détermination des niveaux d'expression de gènes dans un échantillon de sang dudit individu, dans lequel lesdits gènes comprennent un ou plusieurs gènes choisis à partir de PSMC4 ; HSPA8 ; SKP1A ; PSMC4 et HSPA8 ; PSMC4 et SKP1A ; HSPA8 et SKP1A ; et PSMC4, HSPA8 et SKP1A.

10. Procédé selon l'une quelconque des revendications 1 ou 6, dans lequel ladite analyse statistique est basée sur un modèle linéaire général, tel qu'un modèle de régression logistique.

11. Procédé selon la revendication 10, dans lequel ledit profil d'expression représentant le niveau d'expression normalisé de chacun desdits gènes dans ledit échantillon de sang est soumis à la formule $P = e^N/(1+e^N)$,
dans laquelle
N représente la somme pondérée des logarithmes naturels des niveaux d'expression normalisés desdits gènes, avec l'ajout d'une constante ; et
P correspond à la probabilité que l'individu testé a la maladie de Parkinson.

12. Procédé selon la revendication 11, dans lequel

    i. ledit profil d'expression représente les niveaux d'expression normalisés des gènes ALDH1A1, PSMC4, HSPA8, SKP1A, HIP2 et EGLN1 dans un échantillon de sang dudit individu, et ledit profil d'expression est soumis à la formule :

$$P = e^N/(1+e^N),$$

    dans laquelle $N = -2.078 + \sum_{i=1-6}(B_i \, 10 \, \ln(Gene\_exp_i))$ ; chaque i dans ladite formule indiquant un gène i différent pris parmi les six gènes suivants : ALDH1A1, PSMC4, HSPA8, SKP1A, HIP2 et EGLN1 ; $B_i$ est la valeur du coefficient de régression dudit gène i ; $Gene\_exp_i$ est le niveau d'expression relative dudit gène i chez ledit individu ; B(ALDH1A1) est égal à -0.220 ; B(PSMC4) est égal à -0.306 ; B(HSPA8) est égal à 0.435 ; B(SKP1A) est égal à -0.261 ; B(HIP2) est égal à 0.242 ; B(EGLN1) est égal à -0.190 ; et P correspond à la probabilité que l'individu testé a la maladie de Parkinson;
    ii. ledit profil d'expression représente les niveaux d'expression normalisés des gènes ALDH1A1, PSMC4, HSPA8, SKP1A et HIP2 dans un échantillon de sang dudit individu, et ledit profil d'expression est soumis à la formule :

$$P = e^N/(1+e^N),$$

    dans laquelle $N = -0.475 + \sum_{i=1-5}(B_i \, 10 \, \ln(Gene\_exp_i))$ ; chaque i dans ladite formule indiquant un gène i différent pris parmi les cinq gènes suivants : ALDH1A1, PSMC4, HSPA8, SKP1A et HIP2 ; $B_i$ est la valeur du coefficient de régression dudit gène i ; Gene_exp$_i$ est le niveau d'expression relative dudit gène i chez ledit individu ; B(ALDH1A1) est égal à -0.191 ; B(PSMC4) est égal à -0.354 ; B(HSPA8) est égal à 0.411 ; B(SKP1A) est égal à -0.236 ; B(HIP2) est égal à 0.204 ; et P correspond à la probabilité que l'individu testé a la maladie de Parkinson ;

iii. ledit profil d'expression représente les niveaux d'expression normalisés des gènes ALDH1A1, PSMC4, HSPA8 et SKP1A dans un échantillon de sang dudit individu, et ledit profil d'expression est soumis à la formule :

$$P = e^N/(1+e^N),$$

dans laquelle N=-0.818+$\sum_{i=1.4}$(B$_i$ 10 ln(Gene_exp$_i$)) ; chaque i dans ladite formule indiquant un gène i différent pris parmi les quatre gènes suivants : ALDH1A1, PSMC4, HSPA8 et SKP1A; B$_i$ est la valeur du coefficient de régression dudit gène i ; Gene_exp$_i$ est le niveau d'expression relative dudit gène i chez ledit individu ; B(ALDH1A1) est égal à -0.178 ; B(PSMC4) est égal à -0.284 ; B(HSPA8) est égal à 0.438 ; B(SKP1A) est égal à -0.182 ; et P correspond à la probabilité que l'individu testé a la maladie de Parkinson ; ou

iv. ledit profil d'expression représente les niveaux d'expression normalisés des gènes ALDH1A1, PSMC4 et HSPA8 dans un échantillon de sang dudit individu, et ledit profil d'expression est soumis à la formule :

$$P = e^N/(1+e^N),$$

dans laquelle N=-0.176+$\sum_{i=1.3}$(B$_i$ 10 ln(Gene_exp$_i$)) ; chaque i dans ladite formule indiquant un gène i différent pris parmi les trois gènes suivants : ALDH1A1, PSMC4 et HSPA8 ; B$_i$ est la valeur du coefficient de régression dudit gène i ; Gene_exp$_i$ est le niveau d'expression relative dudit gène i chez ledit individu ; B(ALDH1A1) est égal à -0.239 ; B(PSMC4) est égal à -0.322 ; B(HSPA8) est égal à 0.435 ; et P correspond à la probabilité que l'individu testé a la maladie de Parkinson.

13. Procédé selon l'une quelconque des revendications 1, 5, et 6, dans lequel l'individu testé n'a pas reçu de thérapie contre la maladie de Parkinson.

14. Kit pour le diagnostic de la maladie de Parkinson (PD) chez un individu testé, comprenant :

(i) des amorces et des réactifs pour l'amplification quantitative en temps réel par PCR et la mesure des niveaux d'expression de gènes, dans lequel les gènes sont ALDH1A1, PSMA2, LAMB2, et HIST1 H3E

(ii) des amorces et des réactifs pour l'amplification quantitative en temps réel par PCR d'au moins un gène de contrôle pour normaliser les niveaux d'expression mesurés dans l'étape (i) pour obtenir des niveaux d'expression normalisés ; et

(iii) des instructions d'utilisation.

15. Kit selon la revendication 14, comprenant en outre une formule basée sur une analyse statistique de profils d'expression connus des gènes mesurés chez des patients atteints de la maladie de Parkinson et chez des individus de contrôle, pour son application aux niveaux d'expression normalisés pour obtenir une valeur correspondant à la probabilité que l'individu testé a la maladie de Parkinson, dans lequel lesdites instructions comprennent une valeur de coupure prédéterminée à laquelle ladite valeur est comparée.

## Fig. 1

## Fig. 2

## Fig. 3A

## Fig. 3B

## Fig. 3C

## Fig. 3D

## Fig. 3E

## Fig. 3F

## Fig. 3G

## Fig. 3H

## Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005067391 A, Grünblatt

### Non-patent literature cited in the description

- **EDNA GRÜNBLATT et al.** Pilot study: peripheral biomarkers for diagnosing sporadic Parkinson's disease. *JOURNAL OF NEURAL TRANSMISSION,* 11 November 2010, vol. 117 (12), 1387-1393
- **ANDERSEN C.L. ; JENSEN J.L. ; ORNTOFT T.F.** Normalization of real-time quantitative reverse transcription-PCR data: a model-based variance estimation approach to identify genes suited for normalization, applied to bladder and colon cancer data sets. *Cancer research,* 2004, vol. 64 (15), 5245-5250
- **BERG D.** Biomarkers for the early detection of Parkinson's and Alzheimer's disease. *Neurodegener Dis.,* 2008, vol. 5 (3-4), 133-136
- **BERG L.** Clinical Dementia Rating (CDR). *Psychopharmacol Bull,* 1988, vol. 24, 637-639
- **BRAAK H. ; MULLER C.M. ; RUB U. ; ACKERMANN H. ; BRATZKE H. ; DE VOS R.A. ; DEL TREDICI K.** Pathology associated with sporadic Parkinson's disease - where does it end?. *Journal of neural transmission,* 2006, vol. 70, 89-97
- **BROOKS D.J.** Technology insight: imaging neurodegeneration in Parkinson's disease. *Nat Clin Pract Neurol.,* 2008, vol. 4 (5), 267-277
- **CIECHANOVER A. ; BRUNDIN P.** The ubiquitin proteasome system in neurodegenerative diseases: sometimes the chicken, sometimes the egg. *Neuron.,* 2003, vol. 40, 427-446
- **DAUER W. ; PRZEDBORSKI S.** Parkinson's disease: mechanisms and models. *Neuron.,* 2003, vol. 39 (6), 889-909
- **DAVIS J.W. ; GRANDINETTI A. ; WASLIEN C.I. ; ROSS G.W. ; WHITE L.R. ; MORENS D.M.** Observations on serum uric acid levels and the risk of idiopathic Parkinson's disease. *American journal of epidemiology,* 1996, vol. 144 (5), 480-484
- **DAWSON T.M. ; DAWSON V.L.** Molecular pathways of neurodegeneration in Parkinson's disease. *Science,* 2003, vol. 302, 819-822

- **DE PRIL R. ; FISCHER D.F. ; ROOS R.A. ; VAN LEEUWEN F.W.** Ubiquitin-conjugating enzyme E2-25K increases aggregate formation and cell death in polyglutamine diseases. *Mol Cell Neurosci.,* 2007, vol. 34, 10-19
- **ECKERT T. ; TANG C. ; EIDELBERG D.** Assessment of the progression of Parkinson's disease: a metabolic network approach. *Lancet Neurol.,* 2007, vol. 6 (10), 926-932
- **ELLER M. ; WILLIAMS D.R.** Biological fluid biomarkers in neurodegenerative parkinsonism. *Nature reviews,* 2009, vol. 5 (10), 561-570
- UPDRS Development Committee. Unified Parkinson's disease rating scale. **FAHN S. ; ELTON R.** Recent Developments in Parkinson's Disease. Macmillan, 1987, 153-167
- **FEARNLEY J.M. ; LEES A.J.** Ageing and Parkinson's disease: substantia nigra regional selectivity. *Brain,* 1991, vol. 114 (5), 2283-2301
- **FELDMAN R.M. ; CORRELL C.C. ; KAPLAN K.B. ; DESHAIES R.J.** A complex of Cdc4p, Skplp, and Cdc53p/oullin catalyzes ubiquitination of the phosphorylated CDK inhibitor Siclp. *Cell,* 1997, vol. 91 (2), 221-230
- **FISHMAN-JACOB T. ; REZNICHENKO L. ; YOUDIM M.B. ; MANDEL S.A.** A sporadic Parkinson's disease model via silencing of the ubiquitin-proteasorne/E3-ligase component SKPIA. *J Biol Chem.,* 2009, vol. 284 (47), 32835-32845
- **GRÜNBLATT E. ; MANDEL S. ; JACOB-HIRACH J. ; ZELIGSON S. ; AMARIGLO N. ; RECHAVI G. ; LI J. ; RAVID R. ; ROGGENDORF W. ; RIEDERER P.** Gene expression profiling of parkinsonian substantia nigra pars compacta alterations in ubiquitin-proteasome, heat shock protein, iron and oxidative stress regulated proteins, cell adhosion/cellular matrix and vesicle trafficking genes. *J Neural Transm,* 2004, vol. 111 (12), 1543-1573

- **GRÜNBLATT E. ; MANDEL S. ; MÜLLER T. ; JOST W.H. ; YOUDIM M.B. ; RIEDERER P.** Early diagnosis for Parkinson's disease according to whole blood gene profile. *XVII WFN World congress on Parkinson's Disease and related disorders,* 09 December 2017
- **GRÜNBLATT E. ; BARTL J. ; ZEHETMAYER S. ; RINGEL T.M. ; BAUER P. ; RIEDERER P. ; JACOB C.P.** Gene expression as peripheral biomarkers for sporadic Alzheimer's disease. *J Alzheimers Dis,* 2009, vol. 16, 627-634
- **HAMILTON M.** A rating scale for depression. *J Neurol Neurosurg Psychiatry,* 1960, vol. 23, 56-62
- **HENNECKE G.** Scherzer C.R., RNA biomarkers of Parkinson's disease: developing tools for novel therapies. *Biomarkers Med,* 2008, vol. 475 (2), 41-53
- **HJELLE J.J. ; PETERSEN D.R.** Hepatic aldehyde dehydrogenases and lipid peroxidation. *Pharmacol Biochem Behav.,* 1983, vol. 18 (1), 155-160
- **HOEHN M.M. ; YAHR M.D.** Parkinsonism: onset, progression and mortality. *Neurology,* 1967, vol. 17 (5), 427-442
- **HONG Z. ; SHI M. ; CHUNG K.A. et al.** DJ-1 and alpha-synuclein in human cerebrospinal fluid as biomarkers of Parkinson's disease. *Brain,* 2010, vol. 133, 713-726
- **HUGHES A.J. ; DANIEL S.E. ; KILFORD L. ; LEES A.J.** Accuracy of clinical diagnosis of idiopathic Parkinson's disease: a clinico-pathological study of 100 cases. *Journal of necrology, neurosurgery, and psychiatry,* 1992, vol. 55 (3), 181-184
- **HUGHES A.J. ; DANIEL S.E. ; BEN-SHLOMO Y. ; LEES A.J.** The accuracy of diagnosis of parkinsonian syndromes in a specialist movement disorder service. *Brain,* 2002, vol. 125, 861-870
- **JANKOVIC J. ; RAJPUT A.H. ; MCDERMOTT M.P. ; PERL D.P.** The evolution of diagnosis in early Parkinson disease. Parkinson Study Group. *Archives of neurology,* 2000, vol. 57 (3), 369-372
- **MAXDH G. ; VALLEE B.L.** Human class I alcohol dehydrogenases catalyze the interconversion of alcohols and aldehydes in the metabolism of dopamine. *Biochemistry,* 1986, vol. 25, 7279-7282
- **MCKHANN G. ; DRACHMAN D. ; FOLSTEIN M. ; KATZMAN R. ; PRICE D. ; STADLAN E.M.** Clinical diagnosis of Alzheimer's disease: report of the NTNCDS-ADRDA Work Group under the auspices of Department of Health and Human Services Task Force on Alzheimer's disease. *Neurology,* 1984, vol. 34, 939-944
- **MOLLENHAUER B. ; CULLEN V. ; KAHN I. ; KRASTINS B. ; OUTEIRO T.F. ; PEPIVANI I. ; NG J. ; SCHULZ-SCHAEFFER W. ; KRETZSCHMAR H.A. ; MCLEAN PJ.** Direct quantification of CSF alpha-synuclein by ELISA and first cross-sectional study in patients with neurodegeneration. *Experimental neurology,* 2008, vol. 213 (2), 315-325
- **O'CONNOR D.W. ; POLLITT P.A. ; HYDE J.B. ; FELLOWS J.L. ; MILLER N.D. ; BROOK C.P. ; REISS B.B.** The reliability and validity of the minimental state in a British community survoy. *J Psychiatr Res,* 1989, vol. 23, 87-96
- **SCHERZER C.R. ; EKLUND A.C. ; MORSE L.J. ; LIAO Z. ; LOCASCIO J.J. ; FEFER D. ; SCHWARZSCHILD M.A. ; SCHLOSSMACHER M.G. ; HAUSER M.A. ; VANCE J.M.** Molecular markers of early Parkinson's disease based on gene expression in blood. *Proceedings of the National Academy of Sciences of the United States of America,* 2007, vol. 104 (3), 955-960
- **SCHWARZSCHILD M.A. ; SCHWID S.R ; MAREK K. ; WATTS A. ; LANG A.E. ; OAKES D. ; SHOULSON I. ; ASCHERIO A. ; HYSON C. ; GORBOLD E.** Serum urate as a predictor of clinical and radiographic progression in Parkinson disease. *Archives of neurology,* 2008, vol. 65 (6), 716-723
- **SULLIVAN P.F. ; FAN C. ; PEROU CM.** Evaluating the comparability of gene expression in blood and brain. *Am J Med Genet B Nouropsychiatr Genet,* 2006, vol. 141B (3), 261-268
- **TOLOSA E. ; WEANING G. ; POEWE W.** The diagnosis of Parkinson's disease. *ancet neurology,* 2006, vol. 5 (1), 75-86
- **VANDESOMPELE J. ; DE PRETER K. ; PATTYN F. ; POPPE B. ; VAN ROY N. ; DE PAEPC A. ; SPELEMAN P.** Accurate normalization of real-time quantitative RT-PCR data by geometric averaging of multiple internal control genes. *Genome biology,* 2002, vol. 3 (7
- **WEISSKOPF M.G. ; O'REILLY. E. ; CHEN H. ; SCHWARZSCHILD MA. ; ASCHERIO A.** Plasma urate and risk of Parkinson's disease. *American journal of epidemiology,* 2007, vol. 166 (5), 561-567
- **WOUTERS H. ; VAN GOOL W.A. ; SCHMAND B. ; LINDEBOOM R.** Revising the ADAS-cog for a more accurate assessment of cognitive impairment. *Alzheimer Dis Assoc Disord,* 2008, vol. 22 (3), 236-244
- **ZHANG J. ; SOKAL I. ; PESKIND E.R. ; QUINN J.F. ; JANKOVIO J. ; KENNEY C. ; CHUNG K.A. ; MILLARD S.P. ; MUTT J.G. ; MONTINE T.J.** CSF multianalyte profile distinguishes Alzheimer and Parkinson diseases. *American journal of clinical pathology,* 2008, vol. 129 (4), 526-529

- **ZHENG B. ; LIAO Z. ; LOCASCIO J.J. ; LESNIAK K.A. ; RODERICK S,S. et al.** PGC-1$\alpha$, a potential therapeutic target for early intervention in Parkinson's disease. *Sci Transl Med.,* 2010, vol. 2 (52), 52-73